# FASCICULE DE BREVET EUROPEEN

(11) **EP 2 556 089 B1**
(45) Date de publication et mention de la délivrance du brevet: **16.12.2015**
(21) Numéro de dépôt: 11719347.4
(22) Date de dépôt: 08.04.2011
(51) Int. Cl.: C07K 16/00, C12N 15/11, C12Q 1/68

(54) **HEMI-ANTICORPS A AUTO-ASSEMBLAGE**
SELBSTASSEMBLIERENDE ANTIKÖRPERHÄLFTEN
SELF-ASSEMBLING HALF-ANTIBODIES

(30) Priorité: 09.04.2010 FR 1001516
(43) Date de publication de la demande: 13.02.2013
(73) Titulaire: Commissariat à l'Energie Atomique et aux Energies Alternatives, 75015 Paris (FR); Centre National de la Recherche Scientifique, 75794 Paris Cedex 16 (FR)
(72) Inventeur: UGOLIN, Nicolas, 75002 Paris (FR); FALCK, Caroline, 92140 Clamart (FR); LEFEVRE, Emilie, 91540 Mennecy (FR); CHEVILLARD, Sylvie, 75005 Paris (FR); TISNE-VICROBECK, Carine, 92300 Levallois Perret (FR)
(74) Mandataire: Desaix, Anne
(86) Numéro de dépôt international: PCT/FR2011/050812
(87) Numéro de publication internationale: WO 2011/124869

(56) Documents cités:
- WO-A1-95/05399
- WO-A2-00/67697
- VORNLOCHER ET AL: "Antibody-directed cell-type-specific delivery of siRNA", TRENDS IN MOLECULAR MEDICINE, ELSEVIER CURRENT TRENDS, GB LNKD- DOI:10.1016/J.MOLMED.2005.10.009, vol. 12, no. 1, 1 janvier 2006 (2006-01-01), pages 1-3, XP025229885, ISSN: 1471-4914 [extrait le 2006-01-01]
- MURTOLA MERITA ET AL: "PNA based artificial nucleases displaying catalysis with turnover in the cleavage of a leukemia related RNA model.", ORGANIC & BIOMOLECULAR CHEMISTRY 21 OCT 2008 LNKD- PUBMED:18843415, vol. 6, no. 20, 21 octobre 2008 (2008-10-21), pages 3837-3842, XP002639499, ISSN: 1477-0539

### SEQUENCE LISTING

<110> COMMISARIAT A L'ENERGIE ATOMIQUE (CEA)
<120> HEMI-ANTICORPS A AUTO-ASSEMBLAGE
<130> B08781A_AD/SL/CAL
<140> PCT/FR2011/XXXXXX
   <141> 2011-04-08
<150> FR 10/01516
   <151> 2010-04-09
<160> 11
<170> PatentIn version 3.3
<210> 1
   <211> 15
   <212> DNA
   <213> Artificial
<220>
   <223> Polynucléotide (acide nucléique peptidique)
<400> 1
   gtcccguccc cgcca 15
<210> 2
   <211> 8
   <212> PRT
   <213> Artificial
<220>
   <223> Peptide
<220>
   <221> MISC_FEATURE
   <222> (3)..(3)
   <223> Xaa can be any naturally occuring amino acid
<400> 2
   Leu Pro Xaa Thr Ala Ala Ala Ala
   1               5
<210> 3
   <211> 28
   <212> DNA
   <213> Artificial
<220>
   <223> Polynucléotide
<400> 3
   tcaagtagct tcagaatctt ttcccccc 28
<210> 4
   <211> 15
   <212> DNA
   <213> Artificial
<220>
   <223> Polynucléotide
<400> 4
   ggctagctac aacga 15
<210> 5
   <211> 17
   <212> RNA
   <213> Artificial
<220>
   <223> antisens
<400> 5
   gucccugucg ggcgcca 17
<210> 6
   <211> 18
   <212> RNA
   <213> Artificial
<220>
   <223> antisens
<220>
   <221> misc_feature
   <222> (1)..(19)
   <223> Séquence complémentaire
<400> 6
   cagggacaag cccgcggu 18
<210> 7
   <211> 5
   <212> PRT
   <213> Artificial
<220>
   <223> Peptide
<220>
   <221> misc_feature
   <222> (3)..(3)
   <223> Xaa can be any naturally occurring amino acid
<400> 7
   Leu Pro Xaa Thr Gly
   1               5
<210> 8
   <211> 669
   <212> DNA
   <213> Artificial
<220>
   <223> (I-A) HS-LPGTG : partie variable de la chaîne lourde de l'anticorps anti-HEL (Hen Egg Lysozyme)
<400> 8
   caggtgcagc tgaaagaatc tggtccgggt ctggtggcgc cgtctcagtc tctgtctatt 60
   acctgcaccg tgtctggttt ttctctgacc ggttatggtg tgaactgggt gcgtcagccg 120
   ccgggtaaag gtctggaatg gctgggtatg atttggggtg atggtaacac cgattataac 180
   tctgcgctga aatctcgtct gtctatttct aaagataact ctaaatctca ggtgtttctg 240
   aaaatgaact ctctgcatac cgatgatacc gcgcgttatt attgcgcgcg tgaacgtgat 300
   tatcgtctgg attattgggg tcagggtacc accctgaccg tgtcttctgc gtctaccacc 360
   ccgccgtctg tgtttccgct ggcgccgggt tctgcggcgc agaccaactc tatggtgacc 420
   ctgggttgcc tggtgaaagg ttattttccg gaaccggtga ccgtgacctg gaactctggt 480
   tctctgtctt ctggtgtgca tacctttccg gcggtgctgc agtctgatct gtataccctg 540
   tcttcttctg tgaccgtgcc gtcttctccg cgtccgtctg aaaccgtgac ctgcaacgtg 600
   gcgcatccgg cgtcttctac caaagtggat aaaaaaattg tgccgcgtga ttgcctgccg 660
   ggtaccggt 669
<210> 9
   <211> 657
   <212> DNA
   <213> Artificial
<220>
   <223> (I-B) LS-LPGTG : la partie variable de la chaîne légère de l'anticorps anti-HEL (Hen Egg Lysozyme)
<400> 9
   gatattcaga tgacccagtc tccggcgtct ctgtctgcgt ctgtgggtga aaccgtgacc 60
   attacctgcc gtgcgtctgg taacattcat aactatctgg cgtggtatca gcagaaacag 120
   ggtaaatctc cgcagctgct ggtgtattat accaccaccc tggcggatgg tgtgccgtct 180
   cgtttttctg gttctggttc tggtacccag tattctctga aaattaactc tctgcagccg 240
   gaagattttg gttcttatta ttgccagcat ttttggtcta ccccgcgtac ctttggtggt 300
   ggtaccaaac tggaaattaa acgtgcggat gcggcgccga ccgtgtctat ttttccgccg 360
   tcttctgaac agctgacctc tggtggtgcg tctgtggtgt gctttctgaa caacttttat 420
   ccgaaagata ttaacgtgaa atggaaaatt gatggttctg aacgtcagaa cggtgtgctg 480
   aactcttgga ccgatcagga ttctaaagat tctacctatt ctatgtcttc taccctgacc 540
   ctgaccaaag atgaatatga acgtcataac tcttatacct gcgaagcgac ccataaaacc 600
   tctacctctc cgattgtgaa atcttttaac cgtaacgaat gcctgccggg taccggt 657
<210> 10
   <211> 18
   <212> RNA
   <213> Artificial
<220>
   <223> ARN "Primer Binding Sequence" (ou PBS) du HIV-1
<400> 10
   uggcgcccga acagggac 18
<210> 11
   <211> 63
   <212> DNA
   <213> Artificial
<220>
   <223> CCR5-WT (775-837)
<400> 11
   agctctcatt ttccatacag tcagtatcaa ttctggaaga atttccagac attaaagata 60
   gtc 63

## Description

L'invention porte sur de nouvelles molécules chimériques, appelées « hémi-anticorps », capables de s'auto-assembler pour former un site de reconnaissance d'un épitope. Un hémi-anticorps est constitué d'au moins deux molécules chimériques A et B, comprenant chacune un domaine polypeptidique caractéristique d'un domaine variable (ou DV) d'une chaîne lourde ou d'une chaîne légère d'un anticorps, et un domaine nucléotidique, le domaine nucléotidique de A et celui de B étant capables de s'apparier en une structure double-brin, par exemple en milieu hydrique. Sur ces molécules chimériques peuvent être greffées des séquences nucléiques biologiquement actives permettant par exemple d'empêcher l'expression de gènes cibles à l'intérieur d'une cellule de mammifère humain ou non humain.

L'invention concerne également diverses applications de ces hémi-anticorps, et en particulier l'utilisation de ces hémi-anticorps pour des applications de traitement thérapeutique ou de diagnostic *in vitro* et plus généralement de détection de molécules biologiques.

Ces hémi-anticorps présentent un potentiel important d'une part pour le traitement de maladies génétiques ou de cancers et d'autre part pour le traitement de maladies infectieuses. Dans le cadre de certaines maladies génétiques, telles que la myopathie, l'utilisation d'une telle molécule chimérique peut permettre d'empêcher l'expression de gènes mutés au niveau de la traduction, afin de diminuer la présence de protéines défectueuses, à l'origine de la maladie. Dans le domaine de la lutte contre les cancers, cette molécule chimérique peut permettre d'inactiver des protéines intra-cellulaires. Au niveau de maladies infectieuses (en particulier les maladies virales et les infections bactériennes), cette molécule chimérique peut également permettre de bloquer l'expression de gènes de l'agent pathogène qui conditionnent spécifiquement soit des activités primordiales de l'agent pathogène (par exemple, la rétro-transcription chez un rétrovirus), soit des résistances à des traitements existants (par exemple, la résistance des bactéries aux antibiotiques).

Les acides nucléiques, ou analogues d'acides nucléiques, antisens ou possédant une activité ribo- ou desoxyribo-nucléase, sont utilisés pour bloquer l'expression de gènes, que ce soit au niveau de la réplication, de la transcription, ou de la traduction. Parce qu'elles peuvent agir directement sur une séquence ciblée, ces séquences présentent un potentiel important pour le traitement de maladies génétiques, de maladies infectieuses, et dans la lutte contre des mécanismes de résistance (par exemple la résistance des bactéries aux antibiotiques).

Toutefois, l'utilisation de séquences nucléotidiques ou d'analogues en tant que médicament se voit très largement limitée, d'une part par la nécessité d'adresser spécifiquement ces séquences à la cellule cible et, d'autre part par la faible efficacité de passage de ces séquences à travers la membrane cellulaire.

Afin d'améliorer leur efficacité de passage à travers la membrane cellulaire, ces séquences nucléotidiques ou analogues peuvent être modifiés par l'ajout, en 5' ou en 3', de fonctions ou des groupements chimiques lipophiles (Manoharan *et al.,* 1995; Pfeiffer and Höök, 2004) ou de courts peptides, constitués généralement de 1 à 5 résidus d'acides bêta-aminés (c'est-à-dire d'acides aminés dans lesquels le groupe amine est en position bêta (β) par rapport au groupe carboxylate). Cette modification permet de faciliter le passage des acides nucléiques, à travers la membrane plasmique, comme cela a été démontré sur cultures cellulaires (Tripathi *et al.,* 2005).

Bien que facilitant le passage de la membrane plasmique, la simple addition d'un groupement hydrophobe à l'extrémité des séquences nucléiques ne permet cependant pas d'utiliser ces dernières dans le cadre de traitements thérapeutiques, en raison du caractère non spécifique des groupements hydrophobes. En effet, les séquences d'acide nucléique ainsi modifiées peuvent se retrouver, dans leur grande majorité, absorbées de manière aspécifique par diverses cellules avant d'atteindre la cible, diminuant ainsi l'effet du traitement.

Un adressage spécifique de ces molécules vers la cible est donc nécessaire.

Pour palier à ces problèmes d'adressage quelles que soient les conditions du milieu, les inventeurs ont mis au point des molécules chimériques comprenant un domaine nucléique, par exemple de l'ADN, et un domaine polypeptidique caractéristique d'un fragment d'anticorps comprenant un site de reconnaissance d'un épitope.

Ces molécules chimériques acide nucléique/Anticorps (par exemple ADN/Anticorps ou ADN/AC) peuvent être utilisées pour marquer et amplifier les marquages de protéines par exemple à la surface d'une cellule, ou encore pour fonctionnaliser une surface par des anticorps dans le cadre de la réalisation de Biopuces.

Il existe, dans l'art antérieur, des molécules chimériques anticorps/acide nucléique (FR286006; FR2882563 ; FR2882564), qui présentent toutefois des inconvénients :
a) le mécanisme de pénétration à l'intérieur d'une cellule est basé sur l'endocytose ou pinocytose cellulaire, ce qui implique une interaction non covalente entre l'acide nucléique et l'anticorps, de manière à ce que le changement de pH après fusion des vésicules d'endocytose avec des vésicules basiques ou acides intra-cellulaires rompe les interactions non covalentes et sépare les acides nucléiques des anticorps. Ce mode d'adressage et d'absorption fait intervenir une activité cellulaire et n'est donc pas indépendant de l'état de la cellule. Ce mode de réalisation sera peu ou pas efficace sur des modèles cellulaires faisant peu ou ne faisant pas d'endocytose.
b) Les séquences d'acide nucléique qui sont déjà utilisées dans les molécules anticorps/acide nucléique sont généralement de type antisens et éventuellement micro ARN (Mi-RNA) à destination des systèmes protéosomes ; elles n'ont pas d'activité enzymatique propre (Baulcombe, 2002).
c) De plus, le greffage de séquences nucléotidiques sur des anticorps déjà constitués est limité d'une part par la faible reproductibilité du site de greffage sur l'anticorps (Fan *et al.,* 2008) et d'autre part par le maintien du site de reconnaissance de l'anticorps ; il existe en effet une probabilité importante pour que la fonctionnalisation par l'acide nucléique se fasse dans le site actif de l'anticorps, influençant ainsi négativement son activité.
d) La fabrication d'anticorps à partir de séquences clonées reste très difficile à obtenir. Un anticorps est un assemblage complexe de chaînes polypeptidiques reliées par des ponts disulfures. C'est aussi une protéine relativement lourde (en moyenne 150 kDa) et dont la fabrication par des méthodes de clonage et d'expression recombinante *in vitro* reste difficile. En effet, une fois les chaînes produites, l'étape de reconstitution par appariement entre ces chaînes légères et lourdes et l'établissement des ponts disulfures corrects restent limitants. Une grande diversité d'appariements incorrects se forme entre les chaînes et, parmi les combinaisons de chaînes lourdes/légères obtenues, une grande partie n'est pas fonctionnelle en raison d'un mauvais repliement de ces chaînes.

Vornlocher et al. (Trends in Molecular Medicine, 2006, 12(1), 1-3) ont décrit des complexes de fragments Fab d'anticorps-protamine associés à un siARN, dans lesquels la protamine est génétiquement fusionnée à la chaîne lourde d'un fragment Fab d'anticorps (F105) dirigé contre la protéine gp160 du VIH et le siARN est lié au complexe F105-protamine avec une stoechiométrie de 1:6.

La demande internationale WO00/67697 décrit des conjugués pour le transfert d'une molécule d'acide nucléique étranger dans une cellule comprenant une molécule d'acide nucléique, un domaine de translocation et un anticorps spécifique d'un antigène de surface cellulaire.

Murtola et al. (Org. Biomol. Chem., 2008, 6, 3837-3842) ont décrit des nucléases artificielles basées sur des PNAs (PNAzymes) comprenant 2 PNAs reliés entre eux par une molécule d'acide diaminopropionique (Dap) capables de cliver un ARN cible.

Pour palier à ces différents inconvénients, les inventeurs ont mis au point des molécules chimériques particulières, appelées «hémi-anticorps» (ou « HAC »), qui comprennent des polypeptides caractéristiques de fragments des parties variables d'anticorps, et des acides nucléiques (ou des analogues) capables de s'auto-assembler pour reformer un site de reconnaissance d'un anticorps fonctionnel en milieu hydrique, y compris en milieu réducteur.

La délivrance de séquences nucléiques d'intérêt (en particulier de séquences nucléiques biologiquement actives) à l'intérieur de cellules cibles, via les molécules chimères mises au point par les inventeurs, est en partie indépendante du mécanisme d'endocytose et/ou de pinocytose et permet donc de cibler n'importe quelle cellule indépendamment de son métabolisme.

Ainsi, l'invention a pour objet une molécule chimérique appelée « hémi-anticorps », caractérisée en ce qu'elle comprend ou consiste en deux molécules chimériques A et B, comprenant ou consistant chacune en :
(i) un domaine polypeptidique caractéristique d'un domaine variable (ou DV) d'une chaîne lourde ou d'une chaîne légère d'un anticorps, ce domaine polypeptidique étant positionné à une extrémité dans les molécules chimériques A et B, le domaine polypeptidique (i) d'une des deux molécules chimériques, A ou B, étant caractéristique d'un DV d'une chaîne légère d'un anticorps et le domaine polypeptidique (i) de l'autre molécule chimérique, respectivement B ou A, étant caractéristique d'un DV d'une chaîne lourde d'un anticorps (le même anticorps ou un anticorps distinct); et
(ii) un domaine nucléotidique (ou domaine structural) comprenant ou consistant en un polynucléotide, en particulier un ADN ou un ARN, ou un analogue d'un polynucléotide, en particulier un acide nucléique peptidique (ANP), un acide nucléique bloqué (ou LNA ; Locked Nucleic Acid), un acide nucléique méthylphosphonate, ou un acide nucléique thioate (en particulier un acide nucléique phosphorothioate ou phosphorodithioate), le domaine nucléotidique de A et celui de B étant capables de s'apparier en une structure double-brin, par exemple en milieu hydrique, en particulier en milieu réducteur ; et
(iii) le cas échéant, une ou plusieurs molécule (s) de liaison (ou linker(s)) en particulier un linker liant les domaines (i) et (ii) d'une molécule chimérique A ou B, ledit ou lesdits linker(s) comprenant ou consistant en une chaîne hydrogéno-carbonée (ou squelette) comportant de préférence 1 à 20 carbones, les domaines (i) et (ii) de chacune des molécules chimériques A et B étant liés par une liaison covalente, par exemple par l'intermédiaire d'un groupement NH₂, en particulier un groupement NH₂ d'un linker.

Sauf indication contraire, chaque mode de réalisation particulier présenté dans la présente demande peut être combiné indépendamment avec un ou plusieurs autres modes de réalisation particuliers présentés dans la demande.

L'hémi-anticorps de l'invention est capable de s'auto-assembler, en particulier en milieu hydrique ou aqueux, y compris en milieu réducteur, par l'intermédiaire du domaine (ii) de la molécule chimérique A et de celui de la molécule chimérique B, qui s'apparient en une structure double brin.

Le terme « chimérique » désigne ici une molécule qui associe plusieurs domaines, d'au moins deux types différents par leur fonction et/ou par leur nature, au moins deux de ces domaines étant de nature distincte, l'un étant de nature polypeptidique et l'autre étant de nature nucléotidique.

Le terme « plusieurs » tel qu'utilisé dans la présente demande signifie deux ou plus de deux (par exemple trois, quatre, cinq, six ou sept).

Par « hémi-anticorps », on entend ici une molécule chimérique particulière, comprenant ou consistant en :
- au moins une composante peptidique caractéristique d'un anticorps, qui comprend ou consiste en un site de reconnaissance (ou paratope) d'un épitope c'est-à-dire capable de se lier à un épitope; et
- une composante nucléotidique (par exemple une structure nucléotidique double-brin), qui stabilise la molécule chimérique.

Par « domaine variable » (ou « DV »), on entend, au sens de la présente demande, un polypeptide qui constitue, une partie du site de reconnaissance d'un épitope, c'est-à-dire un polypeptide impliqué dans la reconnaissance d'un épitope et dans l'interaction avec ledit épitope. Dans un mode de réalisation particulier de l'invention, ledit DV comprend ou consiste en :
- le domaine variable V_{H} porté par la chaîne lourde d'un anticorps (c'est-à-dire un domaine variable codé par un gène comportant des segments V (variables), J (de jonction) et D (de diversité)) ; ou
- le domaine variable V_{L} porté par la chaîne légère d'un anticorps (c'est-à-dire un domaine variable codé par un gène comportant des segments V et J mais aucun segment D) ; ou
- une ou plusieurs portion(s) de V_{H} ou V_{L,} , en particulier une ou plusieurs portion(s) de V_{H} ou V_{L} capable(s) de se lier audit épitope, ledit DV conservant la capacité de V_{H} ou V_{L} à interagir avec ledit épitope.

A titre d'exemple, dans un mode de réalisation particulier de l'invention, par « portion(s) » de V_{H} ou V_{L} on entend une ou plusieurs (en particulier deux, trois, voire quatre) régions hypervariables, également appelées régions déterminant la complémentarité ou CDRs (de l'anglais « complementary determining régions »), et en particulier tous les CDRs, d'un domaine V_{H} ou V_{L}. Lesdites régions CDRs sont le cas échéant utilisées en combinaison avec les fragments non variables (frameworks 1, 2...) présents dans les domaines variables natifs des anticorps.

Par domaine polypeptidique « caractéristique d'un DV », on entend ici un domaine polypeptidique présentant les propriétés d'un domaine DV, c'est-à-dire un domaine polypeptidique impliqué dans la reconnaissance d'un épitope et dans la liaison audit épitope. Ledit domaine polypeptidique peut être d'origine synthétique ou être issu ou dérivé d'un DV d'un anticorps donné (par exemple un anticorps humain ou humanisé), en particulier ledit domaine polypeptidique peut comprendre ou consister en un DV d'un anticorps donné (par exemple un anticorps humain ou humanisé), ou consister en une séquence obtenue par insertion, délétion ou substitution de résidus d'acides aminés, par exemple un, deux, trois, quatre, ou cinq résidus, dans la séquence d'un DV d'un anticorps donné.

Selon un mode de réalisation particulier, ledit domaine polypeptidique dérive d'un DV obtenu par substitution de résidus d'acides aminés, par exemple un, deux, trois, quatre ou cinq résidus, par un résidu monomère d'un ANP ou d'un acide bêta-aminé (par exemple une bêta-alanine). Ceci permet notamment d'augmenter les possibilités de reconnaissance dudit DV.

Lorsque les molécules chimériques A et B d'un hémi-anticorps sont assemblées (par l'intermédiaire de leur domaine nucléotidique (ii) respectif), le domaine polypeptidique (i) de A et celui de B constituent un site de reconnaissance d'un épitope, capable d'interagir (de préférence spécifiquement) avec un épitope.

Un hémi-anticorps de l'invention peut être obtenu en recombinant des fragments de différents anticorps et en particulier des domaines DV, ou leurs parties actives dans la reconnaissance d'un épitope, issus ou dérivés de différents anticorps, lesdits différents anticorps pouvant provenir ou non de la même espèce de mammifère humain ou non humain. Dans un mode de réalisation particulier de l'invention, un hémi-anticorps comprend au moins deux DV issus ou dérivés d'anticorps donnés (d'un seul et même anticorps ou d'anticorps distincts), en particulier un DV positionné en N-terminal de la molécule chimérique A et un DV positionné en N-terminal de la molécule chimérique B, qui en s'assemblant et en se structurant, forment un site de reconnaissance d'un épitope.

Dans un mode de réalisation particulier de l'invention, le domaine (i) de la molécule chimérique A ou B d'un hémi-anticorps de l'invention comprend ou consiste en le domaine variable V_{H} d'un anticorps ou une ou plusieurs portion(s) du domaine V_{H} d'un anticorps et le domaine (i) de l'autre molécule chimérique dudit hémi-anticorps (respectivement B ou A) comprend ou consiste en le domaine variable V_{L} du même anticorps ou d'un anticorps distinct, ou une ou plusieurs portion(s) de ce domaine V_{L} (lesdites portions de V_{H} ou V_{L} étant telles que définies dans la demande).

Dans un mode de réalisation particulier de l'invention, les domaines (i) des molécules A et B d'un hémi-anticorps sont humanisés.

Le terme « au moins x » élément(s) signifie, dans la présente demande, x élément(s) ou plus de x élément(s), par exemple deux, trois ou quatre éléments.

Par « acide nucléique phosphonate » ou « acide nucléique thioate », on entend ici des acides nucléiques modifiés, dans lesquels des phosphores sont bloqués (c'est-à-dire estérifies par du méthanol) ou substitués par du soufre.

Un domaine polypeptidique (i) peut être caractéristique d'un DV d'une chaîne lourde ou d'une chaîne légère de n'importe quel anticorps.

Dans un mode de réalisation particulier de l'invention, le domaine polypeptidique (i) de la molécule chimérique A et celui de B sont caractéristiques d'un DV d'un même anticorps ou, au contraire, d'anticorps distincts.

Dans un mode de réalisation particulier de l'invention, les domaines polypeptidiques (i) de A et de B comportent au moins une cystéine permettant d'établir un pont disulfure entre le domaine polypeptidique (i) de A et celui de B, et en particulier au moins une cystéine permettant d'établir un pont disulfure entre le DV de A et celui de B.

Dans un mode de réalisation particulier de l'invention, les domaines polypeptidiques (i) de A et de B sont caractéristiques d'un DV d'un anticorps reconnaissant (de préférence spécifiquement) un épitope d'un antigène intra et/ou extra cellulaire, plus particulièrement d'un antigène de surface, par exemple, d'un antigène exprimé à la surface d'organelles ou un antigène de surface cellulaire ou intracellulaire. Ces antigènes peuvent être notamment d'origine cellulaire, virale, bactérienne ou tumorale.

Dans un mode de réalisation particulier de l'invention, les domaines polypeptidiques (i) de A et de B et en particulier leurs domaines polypeptidique (i) N-terminaux sont caractéristiques de DV d'un ou plusieurs anticorps dirigé(s) contre (c'est-à-dire reconnaissant, de préférence spécifiquement) un antigène présent à la surface de cellules de mammifère, par exemple contre la protéine CD4, ou contre un antigène viral, en particulier contre une protéine d'une enveloppe virale, par exemple la protéine d'enveloppe externe d'un virus VIH (virus de l'immunodéficience humaine), ou contre un antigène bactérien, en particulier contre une protéine d'une enveloppe bactérienne, ou contre une rétrotranscriptase, notamment une rétrotranscriptase de VIH-1 ou VIH-2.

Un domaine polypeptidique (i) consiste de préférence en 10 à 300 résidus d'acides aminés et plus préférablement 20 à 200 ou 40 à 200 résidus d'acides aminés (les bornes inférieures et supérieures de ces gammes de valeur étant incluses).

Dans un mode de réalisation particulier de l'invention, les domaines nucléotidiques (ii) tels que définis dans la présente demande sont des acides nucléiques simple brin.

Dans un mode de réalisation particulier de l'invention, le domaine nucléotidique (ii) de la molécule chimérique A et de celui de la molécule chimérique B d'un hémi-anticorps, qui sont des structures simple brin, s'apparient, en milieu hydrique et en particulier en milieu réducteur, en une structure double brin.

Dans un mode de réalisation particulier de l'invention, les domaines nucléotidiques (ii) de A et B sont choisis parmi un polynucléotide, un ANP, ou un LNP et sont capables de former une structure stable double brin, en particulier une hélice alpha.

Dans un mode de réalisation particulier de l'invention, les séquences des domaines nucléotidiques (ii) des deux molécules chimériques A et B sont complémentaires et antiparallèles, et en particulier sont capables de s'hybrider, de préférence sur toute leur longueur. L'hybridation de ces deux domaines nucléotidiques permet de reconstituer une molécule d'hémi-anticorps, en particulier en milieu hydrique (y compris en milieu réducteur). De par cette hybridation, les domaines peptidiques (i) des molécules A et B d'un hémi-anticorps ainsi auto-assemblé (lesdits domaines peptidiques étant, le cas échéant, liés l'un à l'autre par une liaison labile en milieu réducteur, par exemple un pont disulfure) se trouvent rapprochés l'un de l'autre dans l'espace, ce qui permet de structurer et stabiliser le site de reconnaissance de l'épitope, en particulier en milieu réducteur. Ainsi, même en milieu réducteur, le site de liaison à l'antigène est fonctionnel dans l'hémi-anticorps de l'invention.

Dans un mode de réalisation particulier de l'invention, la taille du domaine (ii) varie de 3 à 100 nucléotides et plus préférablement de 5 à 60 nucléotides (les bornes inférieures et supérieures de ces gammes de valeur étant incluses).

Dans un mode de réalisation particulier de l'invention,
- le domaine nucléotidique (ii) de la molécule chimérique A ou B d'un hémi-anticorps de l'invention comprend ou consiste en la séquence suivante : ATGGTAGAG ; et
- le domaine nucléotidique (ii) de l'autre molécule chimérique de l'hémi-anticorps de l'invention, respectivement B ou A, comprend ou consiste en la séquence suivante : CTCTACCAT.

Dans un mode de réalisation particulier de l'invention,
- le domaine nucléotidique (ii) de la molécule chimérique A ou B d'un hémi-anticorps de l'invention comprend ou consiste en la séquence suivante : CCAGCT; et
- le domaine nucléotidique (ii) de l'autre molécule chimérique de l'hémi-anticorps de l'invention, respectivement B ou A, comprend ou consiste en la séquence suivante : AGCTGG.

Dans un mode de réalisation particulier de l'invention, un « linker » au sens de la présente invention est un espaceur, c'est-à-dire qu'il permet d'éloigner dans l'espace les deux domaines qu'il lie, de façon à ce que ces deux domaines puissent exercer leur(s) fonction(s) de façon optimale.

Dans un mode de réalisation particulier de l'invention, un « linker » au sens de la présente invention comporte de préférence 1 à 20 carbones et comprend ou consiste en un ou plusieurs élément(s) (par exemple un élement) choisi(s) parmi un peptide (ou un polypeptide), un polynucléotide en particulier un polynucléotide tel que défini dans la présente demande (ou un analogue, en particulier tel que défini dans la présente demande), une chaîne polycarbonnée, ethylènediamine, polylysine, bêta-alanine, et un sucre.

Dans un mode de réalisation particulier de l'invention, ledit linker est un peptide ou un polypeptide, qui comprend ou consiste par exemple en une ou plusieurs lysine(s). Ce linker sera dénommé dans la présente demande « linker peptidique ».

Dans un mode de réalisation particulier de l'invention, un linker comprend ou consiste en un acide nucléique, en particulier un ADN ou un ARN et consiste par exemple en une séquence de 5 à 50 nucléotides (les bornes inférieures et supérieures de cette gamme de valeur étant incluses). Ce linker sera dénommé dans la présente demande « linker nucléique ».

Dans un mode de réalisation particulier de l'invention, le linker liant le cas échéant les domaines (i) et (ii) d'une molécule chimérique A et le linker liant le cas échéant les domaines (i) et (ii) d'une molécule chimérique B sont des linkers peptidiques.

Dans un mode de réalisation particulier de l'invention, les domaines (i) et (ii) sont liés par l'intermédiaire d'un linker (par exemple un linker peptidique) dans les deux molécules chimériques A et B d'un hémi-anticorps, le linker présent dans A étant de préférence de même taille que le linker présent dans B. Par exemple le même linker peut lier les domaines (i) et (ii) dans A et dans B.

Dans un mode de réalisation particulier de l'invention, un « linker » au sens de la présente invention, par exemple un linker peptidique ou nucléique, comporte une liaison labile en milieu réducteur (par exemple un pont disulfure) ou comporte à l'une de ses extrémités, un ou plusieurs acide(s) aminé(s) ou nucléotide(s) permettant de former, avec une des deux molécules qu'il lie, une telle liaison.

Dans un mode de réalisation particulier de l'invention, « une liaison labile en milieu réducteur » signifie une liaison labile en milieu réducteur et persistante en milieu non-réducteur, ladite liaison pouvant être covalente (par exemple un pont disulfure) ou non covalente.

Selon un mode de réalisation particulier de l'invention, dans la molécule chimérique A, l'extrémité C-terminale du domaine polypeptidique (i) (par exemple un domaine V_{H} ou V_{L} ou une portion d'un domaine V_{H} ou V_{L}) est liée de façon covalente à l'extrémité 3' d'un acide nucléique (ii) (par exemple un LNA, un acide nucléique méthylphosphonate ou un acide nucléique thioate) ou à l'extrémité C-terminale d'un ANP, par exemple par l'intermédiaire du groupement NH₂ de la chaîne latérale d'une lysine liée à l'extrémité C terminale dudit ANP par une liaison peptidique.

Alternativement ou cumulativement, selon un mode de réalisation particulier dans la molécule chimérique B, l'extrémité C-terminale du domaine polypeptidique (i) (par exemple un domaine V_{H} ou V_{L} ou une portion d'un domaine V_{H} ou V_{L}) est liée de façon covalente à l'extrémité 5' de l'acide nucléique (ii) (par exemple un LNA, un acide nucléique méthylphosphonate ou un acide nucléique thioate) ou à l'extrémité N-terminale d'un ANP, par exemple par l'intermédiaire d'une liaison peptidique ou amide avec l'un des groupements NH₂ d'une lysine, ladite lysine étant liée, par son groupement COOH, à l'extrémité N-terminale dudit ANP.

Dans un mode de réalisation particulier de l'invention, la molécule chimérique A et/ou la molécule chimérique B comprend (comprennent) en outre un domaine étiquette et/ou un domaine biologiquement actif, ledit domaine étant lié soit à l'extrémité libre du domaine nucléotidique (ii) de la molécule chimérique A ou B soit à l'extrémité libre d'un linker lié au domaine nucléotidique (ii) de la molécule chimérique A ou B. La liaison dudit domaine étiquette ou dudit domaine biologiquement actif au domaine nucléotidique (ii) de la molécule chimérique A ou B ou à l'extrémité libre dudit linker s'effectue de préférence par l'intermédiaire d'une liaison labile en milieu réducteur, par exemple un pont disulfure, ou au contraire, par l'intermédiaire d'une liaison persistante en milieu réducteur, par exemple par l'intermédiaire d'une liaison établie avec un acide maléïmide.

Dans un mode de réalisation particulier de l'invention, ce linker a une longueur suffisante pour être traversant dans une membrane (par exemple une membrane plasmique ou une membrane intracellulaire).

Dans un mode de réalisation particulier de l'invention, ce linker est un linker nucléique.

Ainsi, dans ce mode de réalisation particulier, l'hémi-anticorps de l'invention permet de délivrer, dans une cellule cible (en particulier une cellule de mammifère humain ou non humain), un domaine biologiquement actif, permettant d'inhiber (complètement ou partiellement) l'expression de gènes dans ladite cellule cible, indépendamment des mécanismes fonctionnels intrinsèques à ladite cellule. Dans le mode de réalisation particulier utilisant une liaison labile en milieu réducteur, le domaine étiquette et/ou le domaine biologiquement actif est libéré à l'intérieur de la cellule après réduction des ponts disulfure.

Dans un mode de réalisation particulier, le domaine étiquette et/ou le domaine biologiquement actif comprend ou consiste en un polynucléotide ou un analogue comprenant ou consistant en une séquence étiquette (ou séquence TAG) et/ou une séquence biologiquement active permettant de bloquer (complètement ou partiellement) l'expression de gènes cibles (notamment au niveau de la réplication, de la transcription, ou de la traduction), en particulier à l'intérieur d'une cellule, par exemple un polynucléotide ou un analogue choisis parmi les éléments suivants:
- un acide nucléique, en particulier un acide nucléique antisens ou un acide nucléique ayant une activité endonucléase par exemple une activité ribonucléase (RNAse) ou desoxyribonucléase (DNAse) ;
- un ANP, en particulier un ANP antisens ou un ANP ayant une activité endonucléase (ANPzyme), par exemple une activité ribo- ou desoxyribo-nucléase,
- un LNA, un acide nucléique méthylphosphonate, un acide nucléique méthylsulfonate, un acide nucléique ou desoxy-nucléique modifié, un ADNzyme, un ARNzyme ou une ANPzyme, un micro ARN (miRNA), une séquence mixte ANP/acide nucléique ou ANP/peptide, un dimère DNA/ANP-zyme ; et
- une combinaison d'au moins deux de ces éléments.

Alternativement ou cumulativement, dans un mode de réalisation particulier, le domaine étiquette et/ou le domaine biologiquement actif comprend ou consiste en un enchaînement de plusieurs (au moins deux) résidus d'acides aminés successifs, formant la structure d'un peptide ou d'un polypeptide. A titre d'exemple, ledit domaine étiquette et/ou ledit domaine biologiquement actif peut comporter au moins 4 ou 6 résidus d'acides aminés, par exemple 4 à 200, 6 à 100 ou 6 à 50 résidus d'acides aminés.

Un peptide désigne, dans la présente demande, une chaîne de 2 à 20 résidus successifs (en particulier 2, 3, 4, 5, 6, 7, 8, 9, 10, 11, 12, 13, 14, 15, 16, 17, 18, 19 ou 20 résidus), en particulier une chaîne de 5 à 10, de 10 à 15 ou de 15 à 20 résidus d'acides aminés successifs.

Dans le cadre de l'invention, un polypeptide, qui désigne également une protéine ou un fragment d'une protéine, est un enchaînement de plus de 20 (au moins 21) résidus successifs, en particulier une chaîne de 21 à 1000 résidus successifs, de préférence de 21 à 500, 21 à 250 ou 21 à 150 résidus successifs, et plus préférablement de 21 à 50 ou 21 à 100 résidus d'acides aminés successifs.

Cumulativement ou alternativement à la présence du domaine étiquette et/ou du domaine biologiquement actif dans la molécule chimérique A et/ou B, selon un mode de réalisation particulier de l'invention, la molécule chimérique A et/ou la molécule chimérique B comprend (comprennent) en outre un élément lipophile (ou hydrophobe), en particulier une séquence, un groupement chimique ou un composé chimique lipophile, qui comporte de préférence un ou plusieurs groupement(s) et/ou composé(s) aromatique(s), par exemple un ou plusieurs résidu(s) tryptophane, ledit élément lipophile étant de préférence positionné à l'extrémité libre de la molécule chimérique A ou B. Cet élément lipophile est le cas échéant lié à la molécule chimérique A ou B par l'intermédiaire d'une liaison labile en milieu réducteur, par exemple un pont disulfure.

Selon un mode de réalisation particulier de l'invention, ledit ou lesdits ou un desdits élément(s) liphophile(s) peuvent être remplacés par un domaine bêta-peptidique, c'est-à-dire un domaine consistant en ou comprenant un ou plusieurs (par exemple 2, 3, 4, 5 ou 6) résidus d'acides bêta-aminés, par exemple 1 à 5 résidus d'acides bêta-aminés. Ledit domaine bêta-peptidique peut par exemple comprendre ou consister en une chaîne de 2, 3, 4, 5 ou 6 résidus d'acides bêta-aminés successifs. A titre d'exemple d'acide bêta-aminé, on citera la bêta-alanine.

Selon un mode de réalisation particulier, un hémi-anticorps de l'invention est tel que :
- la première molécule chimérique, A ou B, comprend ou consiste en :
   - un domaine polypeptidique (i) tel que défini dans la présente demande, et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande,
   - un domaine nucléotidique (ii) tel que défini dans la présente demande,
   - le cas échéant, un linker (par exemple un linker nucléique), lié, de préférence par l'intermédiaire d'une liaison labile en milieu réducteur, par exemple un pont disulfure ou par l'intermédiaire d'une liaison persistante en milieu réducteur, par exemple par l'intermédiaire d'une liaison établie avec un acide maléïmide, à
   - un domaine étiquette ou un domaine biologiquement actif tel que défini dans la présente demande, et
   - le cas échéant, un élément lipophile tel que défini dans la présente demande ledit élément lipophile étant le cas échéant relié au domaine étiquette ou au domaine biologiquement actif par l'intermédiaire d'une liaison labile en milieu réducteur, par exemple un pont disulfure ou par l'intermédiaire d'un linker (par exemple un linker peptidique ou nucléique) tel que défini dans la présente demande ; et
- la deuxième molécule chimérique, respectivement B ou A, comprend ou consiste en :
   - un domaine polypeptidique (i) tel que défini dans la présente demande, ce domaine et le domaine polypeptidique (i) de la première molécule chimérique étant de préférence caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
   et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande, et
   - un domaine nucléotidique (ii) tel que défini dans la présente demande.

Dans un mode de réalisation particulier de l'invention, par « les domaines suivants, positionnés successivement dans l'ordre suivant », on entend « les domaines suivants, liés successivement dans l'ordre suivant ».

Dans un mode de réalisation particulier de l'invention, un hémi-anticorps de l'invention, dénommé HAC_1, est tel que :
- la première molécule chimérique, A ou B, comprend ou consiste en:
   - un domaine polypeptidique (i) tel que défini dans la présente demande, et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande,
   - un domaine nucléotidique (ii) tel que défini dans la présente demande, appelé S1,
   - le cas échéant, un linker (par exemple un linker nucléique) tel que défini dans la présente demande, lié, de préférence par l'intermédiaire d'une liaison labile en milieu réducteur par exemple un pont disulfure, à
   - le cas échéant, un domaine étiquette ou un domaine biologiquement actif tel que défini dans la présente demande, lié, le cas échéant, par exemple, par l'intermédiaire d'une liaison covalente, à
   - le cas échéant, un linker (par exemple un linker nucléique ou peptidique) tel que défini dans la présente demande,
   - un second domaine nucléotidique appelé S3, de préférence lié par l'intermédiaire d'une liaison labile, qui comprend ou consiste en un polynucléotide, en particulier un ADN ou un ARN, ou un analogue d'un polynucléotide, en particulier un ANP, un LNA, un acide nucléique méthylphosphonate, ou un acide nucléique thioate, et qui est capable de s'apparier en une structure double-brin avec un domaine nucléotidique complémentaire appelé S4, par exemple en milieu hydrique, en particulier en milieu réducteur; et
   - le cas échéant, un linker (par exemple un linker peptidique) tel que défini dans la présente demande,
   - le domaine S3 ou ledit linker étant lié à l'extrémité C-terminale d'un second domaine polypeptidique, appelé DV2a, qui est caractéristique d'un DV d'une chaîne lourde ou d'une chaîne légère d'un anticorps, DV2a et le domaine polypeptidique (i) de cette première molécule chimérique (A ou B) étant de préférence caractéristiques de DV d'anticorps distincts, et
   - le cas échéant, un élément lipophile tel que défini dans la présente demande, ledit élément lipophile étant relié à DV2a par l'intermédiaire d'une liaison labile en milieu réducteur, par exemple un pont disulfure ou par l'intermédiaire d'un linker (par exemple un linker nucléique) tel que défini dans la présente demande, ledit linker comportant une liaison labile en milieu réducteur, par exemple un pont disulfure ou étant lié à DV2a par l'intermédiaire d'une liaison labile en milieu réducteur, par exemple un pont disulfure, et
- la deuxième molécule chimérique, respectivement B ou A, comprend ou consiste en:
   - un domaine polypeptidique (i) tel que défini dans la présente demande, ce domaine et le domaine polypeptidique (i) de la première molécule chimérique (de HAC_1) étant de préférence caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
   et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande, et
   - un domaine nucléotidique (ii) tel que défini dans la présente demande, appelé S2, capable de s'apparier en une structure double brin avec S1 en milieu hydrique, en particulier en milieu réducteur.

Dans un mode de réalisation particulier, un hémi-anticorps de l'invention, dénommé HAC_2, est tel que :
- la première molécule chimérique, A ou B, comprend ou consiste en :
   - un domaine polypeptidique (i) tel que défini dans la présente demande, et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande,
   - le domaine nucléotidique (ii) tel que défini dans la présente demande, appelé S5,
   - le cas échéant, un linker (par exemple un linker nucléique ou peptidique) tel que défini dans la présente demande, ledit linker comportant le cas échant une liaison labile en milieu réducteur (par exemple un pont disulfure), ou étant lié par l'intermédiaire d'une liaison labile en milieu réducteur (par exemple un pont disulfure) à,
   - un second domaine nucléotidique appelé S4, de préférence lié par l'intermédiaire d'une liaison labile, qui comprend ou consiste en un polynucléotide, en particulier un ADN ou un ARN, ou un analogue d'un polynucléotide, en particulier un ANP, un LNA, un acide nucléique méthylphosphonate, ou un acide nucléique thioate, et qui est capable de s'apparier en une structure double-brin avec un domaine nucléotidique complémentaire appelé S3, par exemple en milieu hydrique, en particulier en milieu réducteur ; et
   - le cas échéant, un linker (par exemple un linker peptidique) tel que défini dans la présente demande,
   - le domaine S4 ou ledit linker étant lié à l'extrémité C-terminale d'un second domaine polypeptidique, appelé DV2b, qui est caractéristique d'un DV d'une chaîne lourde ou d'une chaîne légère d'un anticorps, DV2b et le domaine polypeptidique (i) de cette première molécule chimérique étant caractéristiques de DV d'anticorps distincts, et
   - le cas échéant, un élément lipophile tel que défini dans la présente demande, ledit élément lipophile étant relié à DV2b par l'intermédiaire d'une liaison labile en milieu réducteur, par exemple un pont disulfure ou par l'intermédiaire d'un linker (par exemple un linker nucléique) tel que défini dans la présente demande, ledit linker comportant une liaison labile en milieu réducteur (par exemple un pont disulfure) ou étant lié à DV2a par l'intermédiaire d'une liaison labile en milieu réducteur (par exemple un pont disulfure); et
- la deuxième molécule chimérique, respectivement B ou A, comprend ou consiste en :
   - un domaine polypeptidique (i) tel que défini dans la présente demande, ce domaine et le domaine polypeptidique (i) de la première molécule chimérique (de HAC_2) étant de préférence caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
   et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande, et
   - un domaine nucléotidique (ii) tel que défini dans la présente demande, appelé S6, capable de s'apparier en une structure double brin avec S5 en milieu hydrique, en particulier en milieu réducteur.
   - Selon un mode de réalisation particulier, dans HAC_1 et dans HAC_2,
      - une liaison labile en milieu réducteur (par exemple un pont disulfure) est présente entre le domaine (ii) et le domaine S3 (ou S4); mais
      - aucune liaison labile en milieu réducteur n'est présente entre les domaines S3 (ou S4) et DV2a (ou DV2b).

HAC_2 peut être utilisé en conjonction avec HAC_1, par exemple administré à un même hôte, en particulier à un mammifère humain ou non humain, HAC_1 et HAC_2 étant administrés séparément (HAC_1 et HAC_2 étant présents dans des compositions, des vésicules ou des particules virales de l'invention distinctes et ce simultanément ou séparément dans le temps).

Différents types de HAC_1 et/ou différents types de HAC_2 peuvent être administrés à un même hôte, en particulier à un mammifère humain ou non humain.

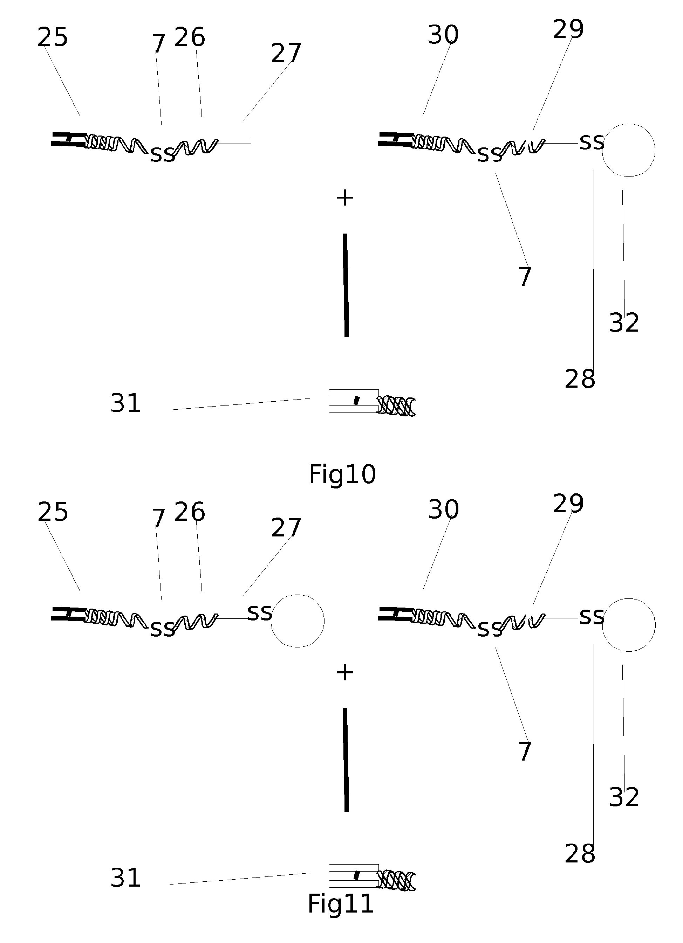

Les HAC_1 et HAC_2 administrés (simultanément ou séparément dans le temps) à un même hôte (en particulier un mammifère humain ou non humain), peuvent également être administrés sous une forme greffée par l'intermédiaire de liaison labile en milieu réducteur sur des nano particules non toxiques, par exemple des nano particules de diamant (voir en particulier l'exemple de la fig. 10). HAC_1 et HAC_2 peuvent en effet être liés (ou greffés) sur une même nano particule ou sur deux nano particules distinctes. Ce greffage est réalisé par l'intermédiaire d'une ou plusieurs liaison(s) labile(s) en milieu réducteur, par exemple une ou plusieurs liaison(s) disulfure, à partir de l'extrémité C-terminale de la molécule chimérique (A ou B) qui comporte le domaine DV2a ou DV2b, et en particulier (lorsque DV2a ou DV2b se trouve en position C-terminale dans HAC_1 ou HAC_2) à partir de l'extrémité N-terminale de la partie DV2a ou DV2b de HAC_1 ou de HAC_2, le cas échéant par l'intermédiaire d'un linker

Ainsi, les systèmes S3-DV2a et S4/DV2b peuvent être délivrés (via HAC_1 et HAC_2 respectivement) dans une même cellule lorsque HAC_1 et HAC_2 ou la ou les nano particule(s) sur lesquels HAC_1 et/ou HAC_2 sont greffés sont internalisées et que les liaisons labiles (par exemple les ponts disulfure) sont clivées.

Les hémi-anticorps HAC_1 et/ou HAC_2 greffés sur une même nano particule peuvent être identiques ou différents, et en particulier différer par leurs domaines S3/DV2a (ou S4-DV2b) et/ou par leurs domaines (i)/(ii). Ainsi, les HAC_1 et/ou HAC_2 greffés sur une même nano particule peuvent comporter :
- un seul type de domaines S3/DV2a (ou S4-DV2b) et un seul type de domaines (i)/(ii) ;
- différents types de domaines S3/DV2a (ou S4-DV2b) et un seul type de domaines (i)/(ii) ;
- un seul type de domaines S3/DV2a (ou S4-DV2b) et différents types de domaines (i)/(ii) ; ou
- différents types de domaines S3/DV2a (ou S4-DV2b) et différents types de domaines (i)/(ii).

Des domaines x/y sont dits « de différents types » au sens de la présente demande lorsqu'ils diffèrent par leur domaine x et/ou par leur domaine y. Selon un mode de réalisation à la fois x et y diffèrent entre deux domaines x/y « de différents types ».

Une nano particule sur laquelle sont greffés des HAC_1 et/ou des HAC_2 comportant différents types de domaines (i)/(ii) permet de cibler des épitopes différents et permet donc généralement de cibler différents types de cellules. En revanche, une nano particule sur laquelle sont greffés des HAC_1 et/ou des HAC_2 comportant un seul type de domaines (i)/(ii) permet de cibler un seul type d'épitope et donc généralement un seul type de cellules.

Dans un mode de réalisation particulier, les nano particules utilisées ont une taille moyenne comprise entre 1 et 100 nanomètres, de préférence entre 1 et 50 nanomètres. Par exemple, leur taille moyenne peut être de 10 nanomètres ou inférieure à 10 nanomètres.

Ainsi, dans les cellules de l'hôte ayant intégré à la fois HAC_1 et HAC_2, un troisième type d'hémi-anticorps, constitué d'une partie de HAC_1 et d'une partie de HAC_2, pourra se former.

A titre d'exemple, dans un mode de réalisation particulier, cet hémi-anticorps, dénommé HAC_3, est tel que :
- la première molécule chimérique, A ou B, comprend ou consiste en :
   - le domaine polypeptidique DV2a, et les domaines suivants, positionnés successivement dans l'ordre suivant, à partir de l'extrémité C-terminale du domaine DV2a:
   - le cas échéant, un linker (par exemple un linker peptidique) tel que défini dans la présente demande,
   - le domaine nucléotidique S3,
   - le cas échéant, un linker (par exemple un linker nucléique ou peptidique) tel que défini dans la présente demande, et
   - le domaine étiquette ou le domaine biologiquement actif présente dans HAC_1 ; et
- la deuxième molécule chimérique, respectivement B ou A, comprend ou consiste en :
   - le domaine polypeptidique DV2b, et les domaines suivants, positionnés successivement dans l'ordre suivant, à partir de l'extrémité C-terminale du domaine DV2b:
   - le cas échéant, un linker (par exemple un linker peptidique) tel que défini dans la présente demande, et
   - le domaine nucléotidique S4.

Dans un mode de réalisation particulier de l'invention, l'un des domaines DV2a et DV2b tels que défini dans la présente demande est caractéristique d'un DV de la chaîne légère d'un anticorps donné et l'autre de ces domaines est caractéristique d'un DV de la chaîne lourde du même anticorps.

Lorsque des hémi-anticorps HAC_1 et des hémi-anticorps HAC_2 sont utilisés et en particulier lorsqu'ils sont présents sur une même particule (en particulier une même nano particule), il peut être avantageux de protéger (temporairement) les parties S3 et S4, par exemple en hybridant ces domaines avec un ARN. Cela permet d'empêcher (temporairement) une éventuelle association entre S3 et S4, de telle sorte que ces deux domaines ne puissent pas s'associer avant d'avoir atteint la cellule cible (en particulier cela permet d'éviter une association prématurée de S3 et S4 lorsque HAC_1 et HAC_2 sont greffés sur une même particule). Les ARN sont ensuite déshybridés ou digérés, ce qui rend alors possible une association entre S3 et S4.

Lorsque l'on souhaite cibler un antigène d'intérêt qui est soluble et en particulier circulant chez un hôte (c'est-à-dire qu'il n'est pas exprimé à la surface d'une cellule), il peut-être intéressant d'utiliser un (ou plusieurs) hémi-anticorps HAC_1 en conjonction avec un (ou plusieurs) hémi-anticorps HAC_2, et de concevoir ces hémi-anticorps de telle sorte que le paratope de HAC_1 et le paratope HAC_2 (c'est-à-dire le site de reconnaissance présent à leur extrémité N-terminale) soient spécifiques du même antigène soluble ou, plus spécifiquement, d'un même épitope de cet antigène soluble.

Selon un mode de réalisation particulier, un hémi-anticorps HAC_1 et un hémi-anticorps HAC_2 sont associés de façon à reconstituer un anticorps complet (présentant deux sites de reconnaissance d'un épitope). De tels anticorps reconstitués peuvent être utilisés notamment à des fins thérapeutiques et/ou diagnostiques et en particulier lorsque le(s) antigène(s) d'intérêt est (sont) soluble(s). A titre d'exemple, on peut utiliser des HAC_1 et HAC_2 dont les paratopes sont spécifiques du même antigène soluble ou, plus spécifiquement, d'un même épitope de cet antigène soluble.

L'invention a également pour objet une molécule chimérique A ou B, telle que définie dans la présente demande.

L'invention a également pour objet une vésicule, en particulier un liposome, et plus particulièrement un liposome constitué de membrane virale, par exemple de membrane de particules du virus VIH-1 ou VIH-2, ou une particule virale, ladite vésicule ou ladite particule virale comportant un ou plusieurs hémi-anticorps tel(s) que défini(s) dans la présente demande et/ou un ou plusieurs molécule(s) chimérique(s) A et/ou B telle(s) que définie(s) dans la présente demande.

L'invention à également pour objet des nano particules (en particulier des nano particules telles que définies dans la présente demande), sur lesquelles un ou plusieurs hémi-anticorps tels que définis dans la présente demande, et en particulier un ou plusieurs hémi-anticorps HAC_1 et/ou un ou plusieurs hémi-anticorps HAC_2 et/ou un ou plusieurs hémi-anticorps HAC_4 (voir ci-après) sont liés (ou greffés), par l'intermédiaire de liaisons labiles en milieu réducteur, par exemple un pont disulfure (cf Fig. 11).

A titre d'exemple, dans un mode de réalisation particulier, la vésicule ou ladite particule virale, la particule ou la nano particule telle que définie dans la présente demande comporte un hémi-anticorps HAC_1 ou un hémi-anticorps HAC_2 tels que définis ci-dessus, et en particulier:
- un hémi-anticorps HAC_1, dans lequel :
   - le domaine polypeptidique (i) des molécules chimériques A et B est de préférence caractéristique d'un même anticorps dirigé contre un premier antigène présent à la surface de cellules de mammifère ou dirigé contre un antigène viral, par exemple contre la protéine d'enveloppe externe d'un virus VIH; et
   - le domaine polypeptidique DV2a est de préférence caractéristique d'un anticorps anti rétro-transcriptase, notamment anti-rétrotranscriptase de VIH-1 ou VIH-2 ; ou
- un hémi-anticorps HAC_2, dans lequel
   - le domaine polypeptidique (i) des molécules chimériques A et B est de préférence caractéristique d'un DV d'un même anticorps dirigé contre un deuxième antigène présent à la surface de cellules de mammifère, par exemple contre la protéine CD4, ou dirigé contre un antigène viral; et
   - le domaine polypeptidique DV2b est de préférence caractéristique d'un DV du même anticorps que le DVdomaine DV2a, DV2a ou DV2b étant caractéristique d'un DV de la chaîne légère d'un anticorps donné, par exemple un anticorps anti rétro-transcriptase, et l'autre domaine, respectivement DV2b ou DV2a, étant caractéristique d'un DV d'une chaîne lourde du même anticorps.

Dans un mode de réalisation particulier de l'invention, deux hémi-anticorps sont utilisés, par exemple un hémi-anticorps HAC_1 et un hémi-anticorps HAC_2 tels que définis dans la présente demande. L'un de ces hémi-anticorps, par exemple HAC_2, peut être présent sous une forme incorporée dans une vésicule, une particule virale ou fixé sur une ou plusieurs particule(s) ou nano particule(s) telles de définies dans la présente demande, l'autre hémi-anticorps, par exemple HAC_1, étant présent en solution. HAC_1 et HAC_2 peuvent être en particulier dirigés respectivement contre un épitope d'un VIH-1 ou VIH-2 et un épitope exprimé à la surface de cellules de mammifères notamment des cellules T, par exemple un épitope de la protéine CD4 des cellules CD4⁺.

L'invention vise également une composition, en particulier une composition pharmaceutique ou thérapeutique, immunologique ou vaccinale, comprenant, consistant en ou consistant essentiellement en :
- un ou plusieurs hémi-anticorps tel(s) que défini(s) dans la présente demande et/ou un ou plusieurs molécule(s) chimérique(s) A et/ou B telle(s) que définie(s) dans la présente demande, ou une ou plusieurs vésicule(s) ou particule(s) virale(s) de l'invention, ou une ou plusieurs particule(s) ou nano particule(s) telle(s) que définie(s) dans la présente demande et
- le cas échéant, un support, un diluant et/ou un véhicule pharmaceutiquement acceptable.

L'expression « consiste essentiellement en » signifie que outre les éléments expressément listés, d'autres ingrédients ou molécules mineur(e)s peuvent être présents dans la composition de l'invention, sans affecter pour autant l'activité des éléments expressément listés.

Une substance ou une combinaison de substances est dite « pharmaceutiquement acceptable » lorsqu'elle est appropriée pour une administration à un hôte à des fins thérapeutiques ou prophylactiques. Elle est donc préférentiellement non toxique pour l'hôte auquel elle est administrée.

Un « hôte » au sens de la présente invention désigne notamment un hôte humain (ou patient) ou un animal non humain, par exemple un mammifère humain ou non humain.

Dans un mode de réalisation particulier, la composition de l'invention comprend, consiste en ou consiste essentiellement en l'hémi-anticorps HAC_1 et/ou l'hémi-anticorps HAC_2 tel(s) que défini(s) dans la présente demande. L'invention a également pour objet, un kit (ou trousse) comprenant :
- un ou plusieurs élément(s) choisi(s) parmi : un hémi-anticorps de l'invention, une molécule chimérique A ou B de l'invention, une vésicule de l'invention, une particule virale de l'invention, une particule ou nano particule telle que définie dans la présente demande et une composition de l'invention,
- et le cas échéant, une notice d'utilisation.

Dans un mode de réalisation particulier, le kit de l'invention comprend l'hémi-anticorps HAC_1 et/ou l'hémi-anticorps HAC_2 tel(s) que défini(s) dans la présente demande.

L'invention a également pour objet, un ou plusieurs élément(s) choisi(s) parmi : un hémi-anticorps de l'invention, une molécule chimérique A ou B de l'invention, une vésicule de l'invention, ou une particule ou nano particule telle que définie dans la présente demande une particule virale de l'invention, une composition de l'invention ou un kit de l'invention, pour une utilisation comme médicament. Lesdits éléments peuvent être notamment utilisés chez un hôte, par exemple un humain ou un mammifère non humain, pour la prévention ou le traitement d'une maladie génétique, par exemple une myopathie, et/ou d'une maladie infectieuse, en particulier d'une maladie virale et plus particulièrement une infection par un lentivirus, par exemple VIH-1 ou VIH-2, et/ou d'une infection bactérienne, par exemple une infection par une bactérie résistante à des antibiotiques et/ou pour la prévention ou le traitement d'un cancer.

L'invention a également pour objet l'utilisation d'un ou plusieurs éléments choisis parmi ceux indiqués ci-dessus, pour la fabrication d'un médicament destiné à prévenir ou traiter les maladies ou infections susmentionnées, chez un hôte, par exemple un humain ou un mammifère non humain.

La présente demande décrit également une méthode pour prévenir ou traiter les maladies ou infections susmentionnées chez un hôte le nécessitant, ladite méthode comprenant ou consistant en une étape d'administration audit hôte d'un ou plusieurs éléments choisis parmi ceux indiqués ci-dessus.

Afin d'augmenter les effets bénéfiques de ces éléments, il est envisageable de procéder à une administration sous la forme de plusieurs administrations successives, répétées à une ou plusieurs occasions, après un intervalle de temps particulier.

La présente demande décrit également l'utilisation d'un ou plusieurs éléments choisis parmi ceux indiqués ci-dessus, pour marquer et/ou amplifier les marquages de protéines, par exemple à la surface d'une cellule, *in vitro, in vivo* et *ex vivo*.

La présente demande décrit également un procédé de synthèse d'un hémi-anticorps tel que défini dans la présente demande. Ce procédé peut comporter une étape dans laquelle le greffage ou la liaison, sur l'extrémité C-terminale du domaine polypeptidique (i) (par exemple, l'extrémité C-terminale d'un domaine V_{H} ou V_{L} ou d'une portion d'un domaine V_{H} ou V_{L}), d'un domaine nucléotidique (ii), par exemple un ANP modifié en N- ou en C-terminal par une lysine, ou un acide nucléique modifié en 5' ou en 3' par un NH₂, se fait par l'action de l'acide 2 mercapto-ethansulfonique ou de l'acide 3 mercaptopropionique, sur une cystéine dudit domaine polypeptidique (i). La lysine ou l'acide nucléique modifié peuvent être remplacés par une bêta-alanine, un éthyl di amine, un NH-CH(CONH2)-(CH2)4NH, ou un NH2(CH2)4(NH2)CH(COOH).

Alternativement ou cumulativement, le procédé de synthèse d'un hémi-anticorps tel que décrit dans la présente demande peut comporter une étape dans laquelle les domaines polypeptidiques (i) dudit hémi-anticorps, qui sont des DV d'anticorps (par exemple des domaines V_{H} ou V_{L} d'anticorps ou comprennent une ou plusieurs portion(s) d'un domaine V_{H} ou V_{L} d'anticorps), sont obtenus soit par digestion d'un ou plusieurs anticorps par une enzyme, par exemple la pepsine, la papaïne, la trypsine, soit par l'expression de polynucléotides codant pour ces DV, dans des systèmes tels que des bactéries, des levures, des cellules d'insectes, des cellules de mammifères, ou par des synthèses *in vitro,* puis par purification.

Alternativement ou cumulativement, pour cloner les domaines polypeptidiques, par exemple des DV tels que définis dans la présente demande, ces domaines (ou leur séquence nucléique à cloner), sont fusionnés à leur extrémité C-terminale (respectivement 3' de la séquence à cloner) avec une séquence peptidique LPXTAAAA (leucine - proline - X - Thréonine - (Alanine)₄, où X est un acide aminé quelconque) respectivement la séquence nucléique codant pour LPXTAAAA. Le domaine nucléotidique à greffer, en particulier l'acide nucléique ou l'ANP à greffer, peut être quand à lui fonctionnalisé à l'une de ses extrémités par un poly-G (poly-glycine), éventuellement par l'intermédiaire d'une lysine, l'extrémité N-terminale du poly-G restant libre. Un domaine polypeptidique (i) (par exemple un DV tel que défini dans la présente demande) et un domaine nucléotidique (ii) (par exemple un acide nucléique (ou polynucléotide) ou un ANP, tels que définis dans la présente demande) sont alors fusionnés sous l'action d'une sortase A.

La séquence LPXTAAAA peut être avantageusement remplacée par la séquence LPXTG (leucine - proline - X - Thréonine - Glycine, où X est un acide aminé quelconque ; SEQ ID NO. 7).

Alternativement ou cumulativement, la fusion entre le DV et le ANP peut être obtenue par catalyse di-MethylSICl₂' diméthyledichloro silane, permettant le greffage d'une fonction amine en C-terminal d'un peptide.

La présente demande décrit aussi la capacité d'amplification de la séquence par PCR au niveau de l'étiquette d'une chimère une fois qu'elle a reconnue un AG fixé sur un support permettant d'identifier et de quantifier l'AG même présent en un seul exemplaire.

L'invention a également pour objet une méthode pour détecter et le cas échéant quantifier un ou plusieurs antigène(s) d'intérêt (plus généralement un ou plusieurs protéines d'intérêt) éventuellement présent(s) dans un échantillon, en particulier un échantillon biologique, ladite méthode comprenant ou consistant en les étapes suivantes :
- la mise en contact des antigènes de l'échantillon avec un ou plusieurs hémi-anticorps tel(s) que défini(s) dans la présente demande et en particulier avec des hémi-anticorps HAC_1 et/ou HAC_2 ;
- la détection, par exemple par PCR, des complexes éventuellement formés entre ledit ou lesdits hémi-anticorps et un ou plusieurs antigène(s) de l'échantillon ;
- le cas échéant, la quantification du ou des antigène(s) ainsi détecté(s).

Un complexe entre un hémi-anticorps et un antigène d'intérêt de l'échantillon se forme par reconnaissance d'un épitope de l'antigène d'intérêt par le site de reconnaissance de l'hémi-anticorps.

Dans un mode de réalisation particulier de l'invention, les antigènes (ou plus généralement les protéines) éventuellement présents dans l'échantillon biologique ont été préalablement fixés sur un support solide, une particule ou tout autre élément permettant d'isoler un complexe formé entre un hémi-anticorps mis en oeuvre et un antigène de l'échantillon du reste de l'échantillon analysé. Alternativement, la méthode peut inclure une première étape consistant en la fixation des antigènes (ou plus généralement des protéines) éventuellement présents dans l'échantillon biologique sur un support solide, une particule ou tout autre élément permettant d'isoler ledit complexe.

Dans un mode de réalisation particulier de l'invention, la détection et le cas échant la quantification d'antigènes présents dans l'échantillon sont réalisées en détectant une séquence particulière présente dans l'hemi-anticorps mis en oeuvre. En particulier, la détection et le cas échant la quantification d'antigènes présents dans l'échantillon peuvent être réalisées en amplifiant, par exemple par PCR, une séquence nucléotidique particulière présente dans l'hemi-anticorps ou un des hémi-anticorps mis en oeuvre (par exemple une séquence nucléotidique particulière présente dans un domaine étiquette dudit hémi-anticorps). Ainsi, sous réserve qu'une quantité suffisante d'hémi-anticorps soit utilisée et que les hémi-anticorps non hybridés aient été éliminés avant de réaliser l'amplification, l'amplification mesurée sera proportionnelle au nombre de molécules de d'hémi-anticorps complexées à un antigène donné (et par conséquent proportionnelle au nombre de molécules à un antigène donné).

Par « échantillon biologique », on entend dans la présente demande un échantillon de sang, de plasma, de sérum, d'urine ou de tout autre fluide susceptible de provenir d'un hôte (en particulier d'un mammifère) humain ou non humain, ou un échantillon de tissu ou de cellule (voire une unique cellule).

L'invention à aussi pour objet l'utilisation d'un (ou plusieurs) hémi-anticorps de l'invention pour la mise en oeuvre d'une méthode d'analyse réalisée à partir d'une puce (ou biopuce) à acide(s) nucléique(s) (en particulier à ADN et/ou ARN et/ou ANP et/ou ALN...) pour réaliser une puces à anticorps ou une puce mixte anticorps/acides nucléiques.

Ainsi, l'invention a également pour objet une seconde méthode pour détecter et le cas échéant quantifier un ou plusieurs antigène(s) d'intérêt (plus généralement une ou plusieurs protéines d'intérêt) éventuellement présent(s) dans un échantillon (en particulier un échantillon biologique), ladite méthode comprenant ou consistant en les étapes suivantes:
a) la mise en contact d'une ou plusieurs unités d'hybridation (ou spots) d'une puce à acide nucléique avec un (ou plusieurs) hémi-anticorps tel que défini dans la présente demande et en particulier avec un hémi-anticorps HAC_1, HAC_2 ou HAC_4, l'hémi-anticorps étant capable de se fixer (en particulier de s'hybrider) à une ou plusieurs unité(s) d'hybridation de ladite puce ;
b) la mise en contact des unités d'hybridation de la puce (ou du moins des unités d'hybridation ayant été mises en contact avec l'hémi-anticorps à l'étape a) avec les protéines ou les antigènes de l'échantillon (ou plus généralement avec l'échantillon)
c) la détection des complexes éventuellement formés entre l'hémi-anticorps fixé sur la puce à l'issue de l'étape b) et un ou plusieurs antigène(s) de l'échantillon ;
d) le cas échéant, la quantification du ou des antigène(s) ainsi détecté(s).

Dans un mode de réalisation particulier de l'invention, l'étape a) est supprimée dans la méthode décrite ci-dessus, la puce mise en oeuvre dans le cadre de cette méthode étant une puce à anticorps ou une puce mixte anticorps/acides nucléiques telle que définie ci-après.

Dans un mode de réalisation particulier de l'invention, ladite méthode d'analyse par biopuce comprend ou consiste en les étapes suivantes :
a) l'étape a) telle que définie ci-dessus ;
b) la mise en contact des unités d'hybridation de la puce (ou du moins des unités d'hybridation ayant été mises en contact avec l'hémi-anticorps à l'étape a) avec l'échantillon ;
c) la détection des complexes éventuellement formés entre l'hémi-anticorps fixé sur la puce à l'issue de l'étape b) et un ou plusieurs antigène(s) de l'échantillon ;
d) la détection des complexes éventuellement formés entre d'une part des acides nucléiques ou des ANP présents sur une ou plusieurs unité(s) d'hybridation de la puce et d'autre part des acides nucléiques complémentaires présents dans l'échantillon ;
e) le cas échéant, la quantification du ou des antigène(s) de l'échantillon détecté(s) à l'étape c) et/ou du ou des acide(s) nucléique(s) de l'échantillon détecté(s) à l'étape d).

L'étape d) peut être réalisée après ou avant l'étape c) ou simultanément à l'étape c).Dans un mode de réalisation particulier de l'invention, les étapes de détection et/ou de quantification, ou certaines étapes de détection et/ou de quantification sont réalisées par amplification par PCR et/ou par microscopie confocale.

Dans un mode de réalisation particulier de l'invention, les étapes a) et b) sont remplacées par les étapes suivantes :
a) un (ou plusieurs) hémi-anticorps tel que défini dans la présente demande et en particulier un hémi-anticorps HAC_1, HAC_2 ou HAC_4, est mis en contact avec les protéines ou les antigènes de l'échantillon (ou plus généralement avec l'échantillon) dans des conditions permettant la formation de complexes protéine / HAC ; puis
b) l'hémi-anticorps de l'étape a) est mis en contact avec une ou plusieurs unités d'hybridation (ou spots) d'une puce à acide nucléique sur laquelle l'hémi-anticorps est capable de se fixer (en particulier de s'hybrider).

Dans un mode de réalisation particulier, à l'étape b), les hémi-anticorps mis en contact avec les unités d'hybridation sont les hémi-anticorps présents sous forme de complexes protéine / HAC.

Dans un mode de réalisation particulier, les puces utilisées dans le cadre de l'invention sont des puces à impédance, et en particulier des puces telles que divulguées dans les demandes WO 2005/036170 et WO 2006/090073.

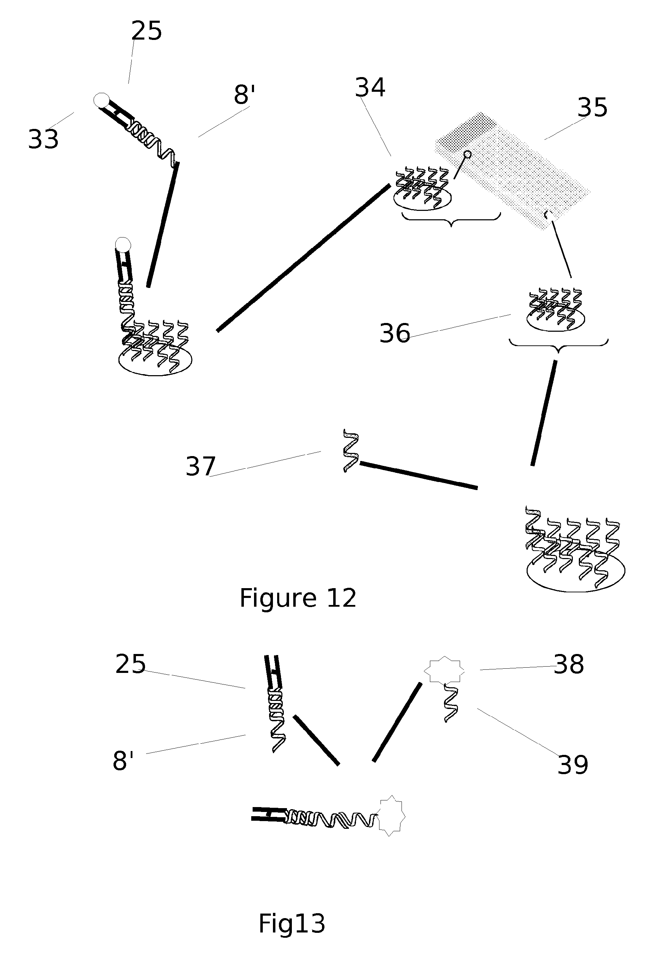

Dans un mode de réalisation particulier de l'invention, le(s) hémi-anticorps ou un des hémi-anticorps mis en oeuvre dans la méthode d'analyse par biopuce selon l'invention est(sont) tel(s) qu'il(s) comporte(nt) un domaine nucléotidique (par exemple un domaine étiquette) complémentaire à une séquence nucléotidique présente sur une ou plusieurs unité(s) d'hybridation de la puce utilisée (voir figure 12).

A titre d'exemple, dans un mode de réalisation particulier de l'invention, l'hémi-anticorps mis en oeuvre dans la méthode d'analyse par biopuce selon l'invention est un hémi-anticorps HAC_4 tel que :
- la première molécule chimérique, A ou B, de cet hémi-anticorps comprend ou consiste en :
   - un domaine polypeptidique (i) tel que défini dans la présente demande, et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande,
   - un domaine nucléotidique (ii) tel que défini dans la présente demande,
   - le cas échéant, un linker (par exemple un linker nucléique), lié, de préférence par l'intermédiaire d'une liaison labile en milieu réducteur (par exemple un pont disulfure) ou par l'intermédiaire d'une liaison persistante en milieu réducteur (par exemple par l'intermédiaire d'une liaison établie avec un acide maléïmide), à un domaine étiquette complémentaire à une séquence nucléotidique d'une unité d'hybridation sur ladite puce (ceci permet la fixation de la molécule chimérique sur l'unité d'hybridation spécifique de la puce, transformant par la même l'unité d'hybridation en une unité de complexation pour la fixation d'une cible par reconnaissance anticorps-antigène ; et
- la deuxième molécule chimérique, respectivement B ou A, de cet hémi-anticorps, comprend ou consiste en:
   - un domaine polypeptidique (i) tel que défini dans la présente demande, ce domaine et le domaine polypeptidique (i) de la première molécule chimérique étant de préférence caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
   et les domaines suivants, positionnés successivement dans l'ordre suivant, à partir de l'extrémité C-terminale du domaine polypeptidique (i):
   - le cas échéant, un linker (par exemple un linker peptidique) tel que défini dans la présente demande, et
   - un domaine nucléotidique (ii) tel que défini dans la présente demande, capable de s'apparier en milieu hydrique (en particulier en milieu réducteur) en une structure double brin avec le domaine nucléotidique (ii) de la première molécule chimérique, respectivement A ou B.

L'invention à aussi pour objet une puce (ou biopuce) telle que définie dans le présente demande, qui comporte une ou plusieurs unités d'hybridation sur lesquelles un (ou plusieurs) hémi-anticorps tel que défini dans la présente demande est complexé (ou lié). Ladite puce est par conséquent dénommée « puce (ou biopuce) à anticorps » (lorsque toutes les unités d'hybridation de ladite puce sont hybridées avec un hémi-anticorps) ou une « puce mixte anticorps/acides nucléiques » (lorsque une ou plusieurs unités d'hybridation de ladite puce ne sont pas hybridées avec un hémi-anticorps mais avec un acide nucléique).

L'invention à également pour objet un kit, en particulier kit approprié pour une utilisation pour détecter et le cas échéant quantifier un ou plusieurs antigène(s) d'intérêt éventuellement présent(s) dans un échantillon, ledit kit comprenant ou consistant en :
a)
   - une puce à acide(s) nucléique(s) ; et
   - un ou plusieurs hémi-anticorps tel(s) que défini(s) dans la présente demande ou une ou plusieurs composition(s) comprenant un ou plusieurs hémi-anticorps selon l'invention; et
   - le cas échéant, une notice d'utilisation ; ou
b)
   - une puce à anticorps ou une puce mixte anticorps/acides nucléiques selon l'invention ; et
   - le cas échéant, une notice d'utilisation.

La présente invention peut donc permettre de fonctionnaliser des unités de complexation d'une puce à acide nucléique, de sorte à conférer auxdites unités de complexation la capacité de reconnaître des antigènes et en particulier épitopes.

De façon similaire, il est possible de fonctionnaliser n'importe quel autre élément présentant une surface, en particulier des particules par des hémi-anticorps selon l'invention, par l'intermédiaire par exemple d'ANP ou d'acides nucléiques complémentaires d'un domaine étiquette présent dans lesdits hémi-anticorps (Cf Fig.13).

Ainsi, l'invention a également pour objet une particule (par exemple une nanoparticule), et en particulier une particule fluorescente ou non fluorescente, caractérisée en ce qu'un ou plusieurs hémi-anticorps tel(s) que défini(s) dans la présente demande et en particulier un ou plusieurs hémi-anticorps HAC_1 et/ou un ou plusieurs hémi-anticorps HAC_4 sont liés à la surface de ladite particule. La liaison hémi-anticorps/particule peut se faire par l'intermédiaire d'un ANP ou d'un acide nucléique comportant une séquence complémentaire à une séquence nucléique dudit hémi-anticorps (par exemple complémentaire au domaine étiquette de l'hémi-anticorps.

Ladite particule peut être par exemple un quantum dot (ou Q-dot).

Ladite particule peut être utilisée notamment pour marquer (*in vivo, ex vivo* ou *in vitro*) des cellules cibles, c'est-à-dire des cellules à la surface desquelles est présent un épitope reconnu par le site de reconnaissance d'un hémi-anticorps selon l'invention.

La présente demande décrit également l'adressage (*in vivo, ex vivo* ou *in vitro*) d'une ou plusieurs (en particulier 2) ANPzyme vers des cellules cibles.

L'ANP catalytique (dénommé ANPzyme dans la présente demande), utilisé dans la présente demande présente la capacité de couper spécifiquement un ADN. Cette ANPzyme comprend ou consiste en un ou plusieurs motif(s) HD (histidine - acide aspartique), ou DH (acide aspartique - histidine), EH (acide glutamique - histidine) ou HE (histidine-acide glutamique). L'utilisation d'une seule ANPzyme permet de réaliser des coupures dans l'ADN alors que l'utilisation de deux ANPzymes de telle sorte que deux séquences contiguës et non complémentaires situées sur les deux brins d'un ADN complémentaire soient coupées permet de couper les deux brins, de la manière d'une enzyme de restriction entraînant une coupure double brin décalée ; l'utilisation de système d'ANPzymes contiguës permet d'introduire des délétions dans l'ADN.

Le ou les systèmes ANPzyme peuvent être apportés à une cellule cible par tout moyen et en particulier en utilisant un ou plusieurs hémi-anticorps de l'invention.

L'invention concerne donc en particulier un hémi-anticorps de l'invention, dans lequel le domaine étiquette et/ou le domaine biologiquement actif comprend ou consiste en une ou plusieurs ANPzyme (en particulier une ou plusieurs ANPzyme comportant un motif HD ou DH). Lorsque deux ANPzymes sont utilisées dans le cadre de l'invention, elles sont préférablement portées par le même hémi-anticorps, qui peut être greffé sur une nano particule non toxique (comme décrit dans la présente demande).

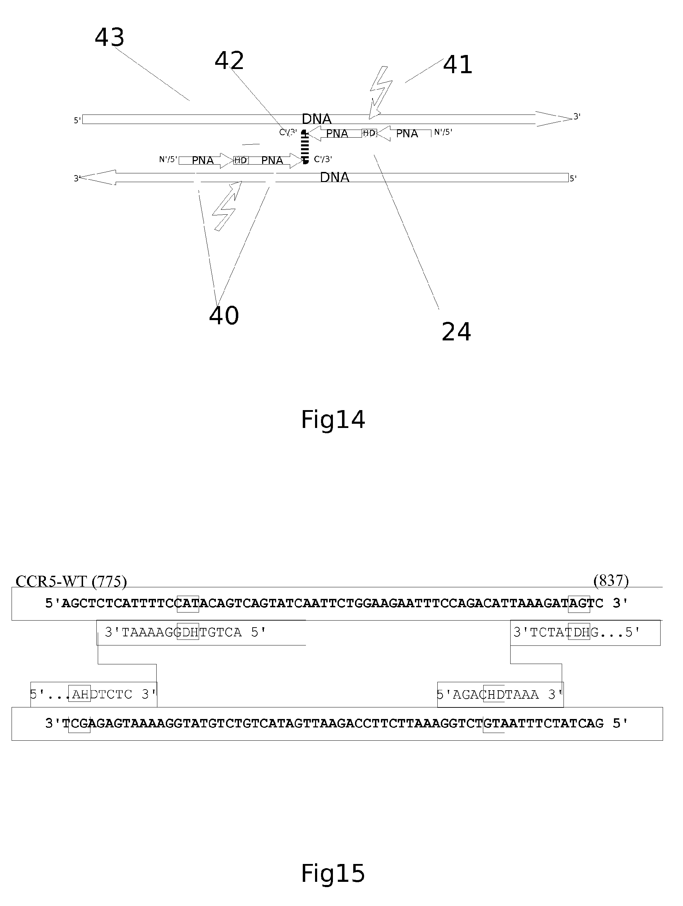

Dans un mode de réalisation particulier, l'invention concerne un hémi-anticorps tel que défini dans la présente demande, dans lequel le domaine étiquette et/ou le domaine biologiquement actif comprend ou consiste en deux ANPzyme en particulier deux ANPzyme à motif HD ou DH, qui sont telles que les deux ANPZYME soit liées par leurs extrémités N-terminale ou éventuellement par leurs extrémités C-terminale par l'intermédiaire d'un même linker (ces deux ANPzyme sont dénommées dans la présente demande « double anpzymtête bêche » ou « DAZtb »), de manière à ce que ces ANPZYME reconnaissent deux séquences contiguës et non complémentaires situées sur les deux brins d'un ADN complémentaire (voir Fig. 14). DAZtb, en s'hybridant à l'ADN, permet de réaliser une coupure double brin décalée entre les deux brins d'une molécule ADN. L'adressage de deux DAZtb par deux molécules chimériques, fixées par exemple à une même nano particule, et comportant le même domaine de DV mais deux DAZtb différents (donc définissant des sites de coupures décalés), encadre une région donnée d'une molécule d'ADN, permettant de réaliser la délétion de cette région.

Cet outil peut notamment être utilisé pour réaliser la délétion de la région de la séquence nucléotidique d'un virus, par exemple d'un virus HIV et en particulier du virus HIV-1 conférant la résistance audit virus (voir Fig. 15) ou la délétion d'une région de la séquence nucléotidique d'une bactérie conférant la résistance à un ou plusieurs antibiotique(s); à titre d'exemple, on peut citer la séquence PBS ou des séquences responsables de la pénétration dans le noyau.

La présente demande décrit également une enzyme ou PNAzyme en tant que telle, formée par une structure comprenant ou consistant en :
- une séquence PNA, reliée à
- une séquence peptidique, reliée à
- une autre séquence PNA,
les deux séquence PNA pouvant être identiques ou différentes.

Tel que décrites dans la présente demande, les séquences PNA sont déterminées pour cibler une séquence d'acide nucléique particulière (par exemple une séquence d'ADN particulière) et s'y apparier.

La présente demande décrit deux structures PNAzyme telles que définies ci-dessus (ces deux PNAzyme pouvant avoir des séquences identiques ou différentes), reliées de telles sortent qu'elles soient tête-bêche, c'est-à-dire reliées par leurs extrémités C-terminales, ou (préférablement) par leurs extrémités N-terminales, de préférence par l'intermédiaire d'un même linker. Une telle double structure PNAzyme est appelée « double PNAzyme tête-bêche ».

Par « séquence peptidique » on entend ici une chaîne d'au moins deux (c'est-à-dire deux ou plus de deux) acides aminés.

Tel que décrite dans la présente demande, la séquence peptidique présente dans une PNAzyme constitue un domaine catalytique d'une enzyme et en particulier d'une nucléase, préférablement d'une DNAse.

Tel que décrite dans la présente demande, ladite séquence peptidique comprend ou consiste en un ou plusieurs motifs DH (acide aspartique - histidine), HD (histidine - acide aspartique), EH (acide glutamique - histidine) ou HE (histidine - acide glutamique).

Une PNAzyme et en particulier une double PNAzyme tête-bêche permet d'opérer, comme avec une enzyme de restriction, une coupure simple brin ou double brin dans un ADN. De plus, l'utilisation deux doubles PNAzymes tête-bêche (donc de 4 PNAzymes) permet la création de délétions dans un ADN double brin, le cas échéant, au moyen de coupures jointives sur deux brins d'ADN complémentaires.

D'autres modes de réalisation particuliers, caractéristiques et avantages de l'invention apparaissent dans les exemples qui suivent ainsi que dans les figures et en particulier dans la figure 12.

### DESCRIPTION DES DESSINS

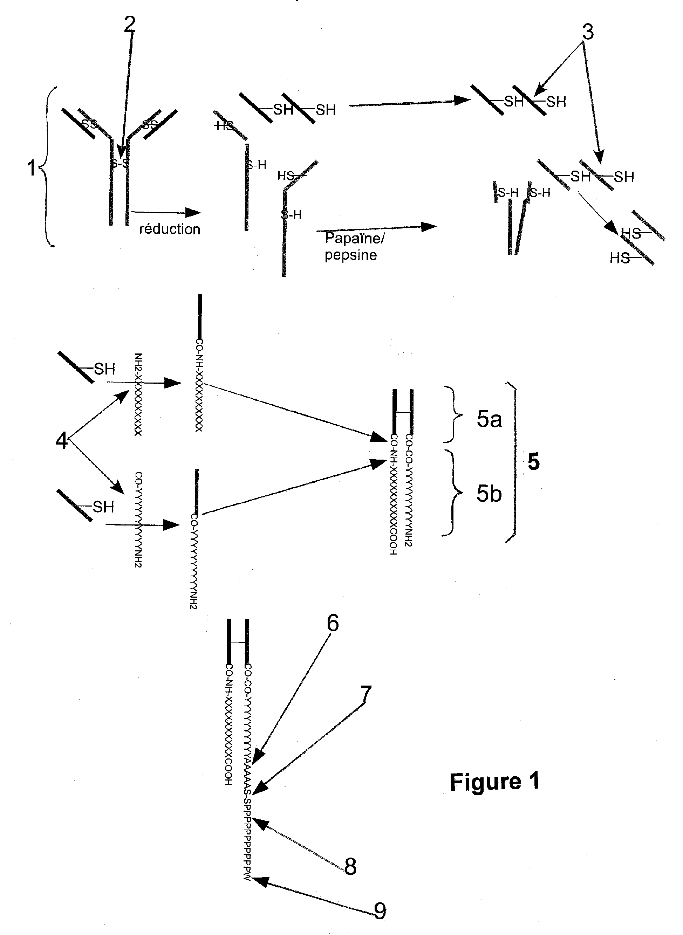

**Figure 1** **: Exemple de synthèse réalisée.** 1 : anticorps natif ; 2 : pont disulfure (ou pont S-S) ; 3 : domaines variables (DV) de chaîne légère et de chaîne lourde ; 4 : domaines nucléotidiques structuraux (ANP structural ou Zipper) ; 5. hémi-anticorps ; 5a : site actif (DV1a+DV1b ; site comportant un site de reconnaissance d'un épitope) de hémi-anticorps structuré ; 5b : acides nucléiques structuraux appariés formant une structure de type zipper ; 6-8 : séquence nucléotidique tag comportant une séquence biologiquement active ; 6 : séquence linker/espaceur ; 7 : pont disulfure labile ; 8 : séquence biologiquement active (ANP,LNA...) ; 9 : élément lipophile (ex :tryptophane).

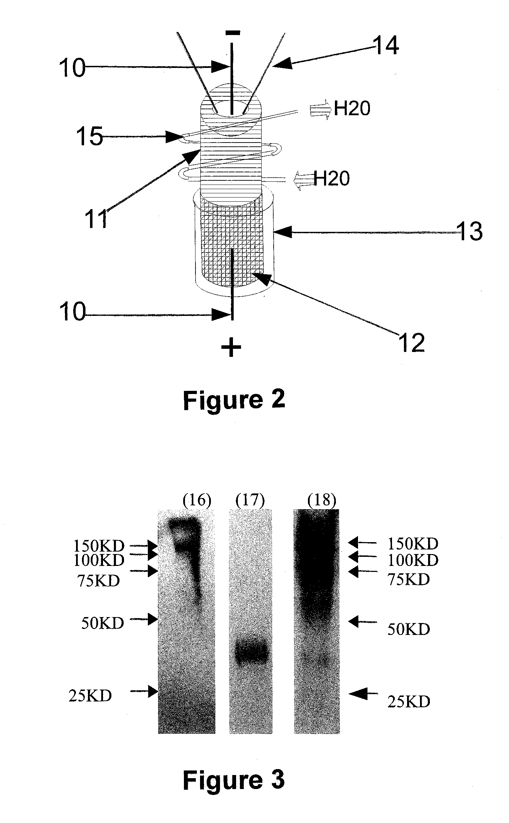

**Figure 2****. Réacteur.** 10 : Electrode ou électrode et gel reliés aux bornes d'un générateur ; 11 : Tube de laiton contenant la solution à réduire ; 12 : Gel duracryl & billes SH ; 13 : Tube en matière plastique (Tycon) isolant électrique ; 14 : Guide d'électrode; 15 : Spirale de refroidissement, Radiateur dans lequel circule de l'eau à une température inférieure à 10°C.
**Figure 3****.** 16: Analyse western-blot Anticorps natifs ; 17 : Analyse western-blot Anticorps dénaturés ; 18 : Analyse western-blot Anticorps électroréduits dans le réacteur laiton /Tycon à 100V, 2mA, 10 sec.
**Figure 4****.** 19: antisens acides nucléique bloquant; 22 : Interaction, mise en évidence par RMN, entre un ANP w-GTCCCGUCCCCGCCA-c ; SEQ ID No. 1 (w : tryptophane ; c : cystéine); 23 : boucle de l'activité RNase ou DNAse d'une DNAzyme ANP/DNAzyme ; 24 : motif DH ou HD d'une boucle enzymatique d'une ANPzyme.
**Figure 5****.** 6 : séquence linker/espaceur ; 7 : pont disulfure labile ; 8 : séquence biologiquement active (ANP, LNA...) ; 25 : HAC_tag (1) ; 26 : ANP structural 1 (S3) ; 27 : DV2a) ; 28 : DV2b) ; 29 : ANP structural 1 (S4) ; 30 : HAC_tag (2) ; 31 : HAC_tag (3)
**Figure 6** : 20 : interaction, mise en évidence par RMN, entre un ANP w-CGCCA-c (w : tryptophane et c : cystéine avec N-terminal 5' et C-terminal 3') ; 21 :Interaction, mise en évidence par RMN, entre un ANP w-GTCCC-c (w : tryptophane et c : cystéine avec N-terminal 5' et C-terminal 3') ; 22 : Interaction, mise en évidence par RMN, entre un ANP w-GTCCCGUCCCCGCCA-c ; SEQ ID No. 1 (w : tryptophane ; c : cystéine).

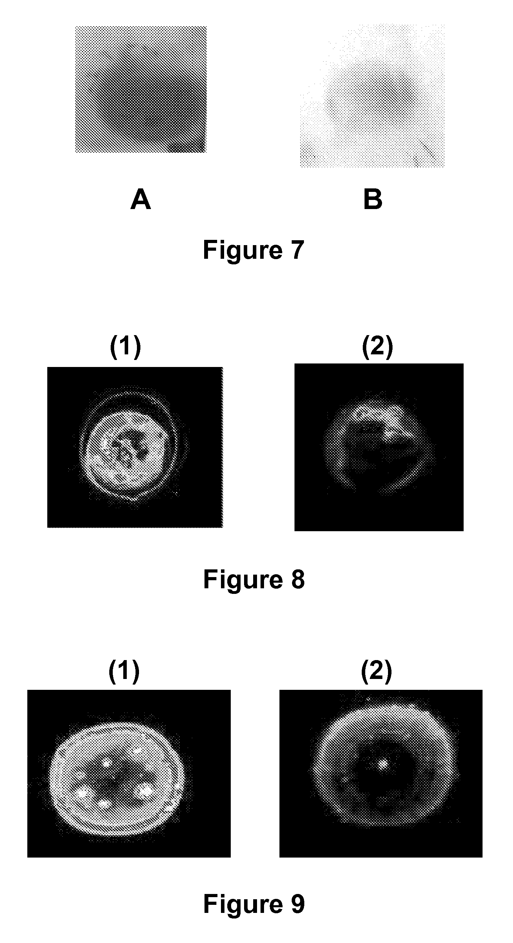

**Figure 7** **: reconnaissance anticorps/antigène sur membrane de nitrocellulose**. A. Anticorps électro-réduits + BSA. B. Chimère + BSA.
**Figure 8** **: Résultats de la fluorescence par marquage sur membrane de nitrocellulose**. (1) Fluorescence du surnageant chimère/BSA sur nitrocellulose ( ; (2) contrôle négatif (sonde fluorescente sur BSA).
**Figure 9****: Résultats de la fluorescence sur les pièges magnétiques.** (1) surnageant des pièges magnétiques / chimères marquées ; (2) contrôle négatif (sonde fluorescente avec pièges magnétiques).
**Figure 10** **: Chimère libre et chimère greffées sur particules.** 7 : pont disulfure labile ; 25 : HAC_tag (1) ; 26 : ANP structural 1 (S3) ; 27 : DV2a) ; 28 : DV2b) ; 29 : ANP structural 1 (S4) ; 30 : HAC_tag (2) ; 31 : HAC_tag (3); 32 : particle.
**Figure 11** **: chimère greffées sur particules.** 7 : pont disulfure labile ; 25 : HAC_tag (1) ; 26 : ANP structural 1 (S3) ; 27 : DV2a) ; 28 : DV2b) ; 29 : ANP structural 1 (S4) ; 30 : HAC_tag (2) ; 31 : HAC_tag (3); 32 : particule.
**Figure 12** **: puces acide nucléique mixte Protéine/acide nucléique.** 8' : séquence étiquette tag (ANP, LNA...) ; 25 : HAC_tag ; 35 : Puce (ANP, LNA, acides nucléiques...); 34 Spot ANP Anti-séquence étiquette ; 36 : Spot ANP Anti-séquence ADN/ARN. 37 : acide nucléique cible.
**Figure 13****: Fonctionalisation particule fluoréscentefluorescente.** 8' : séquence étiquette tag (ANP, LNA...) ; 25 : HAC_tag ; 38 : particule fluorescente ; 39 séquence complémentaire de l'étiquette tag.
**Figure 14****.** 24 : motif enzymatique DH ou HD d'une ANPzyme ; 40 : partie ANP du PNAzyme ; 41 : site de coupure linkers NN ou CC entre ANP, LNA...
complémentaires ; 42 : linker reliant les parties C-terminales de deux ANPZyme tête-bêche; 43 : DNA cible à déléter.

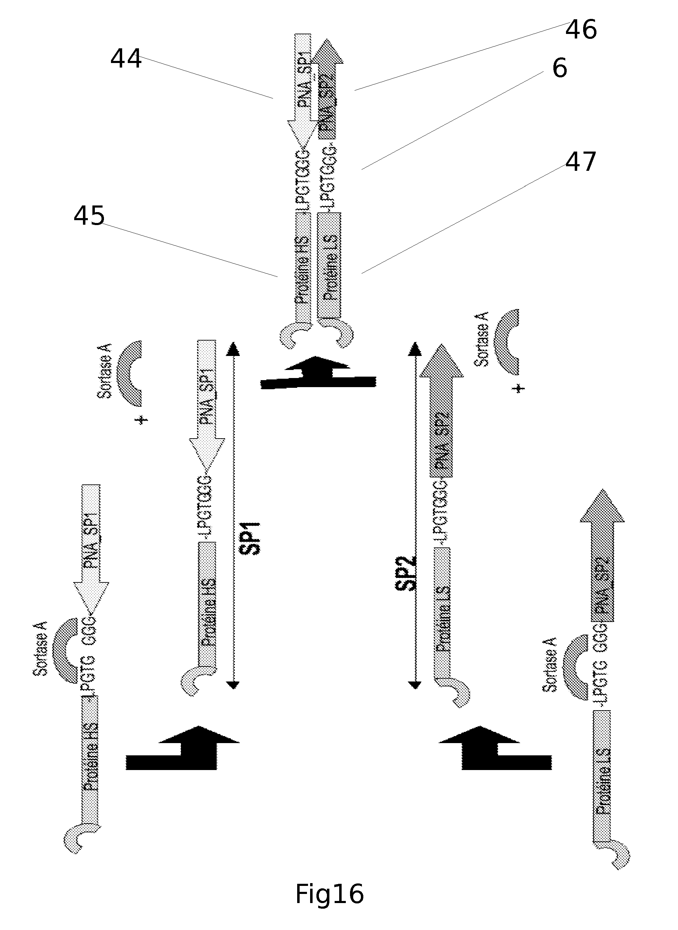

**Figure 16****.** 6 : séquence linker/espaceur ; 44 : ANP structural S1 ; 45 : DVA; 46 : DVB; 47: ANP structural (S2).

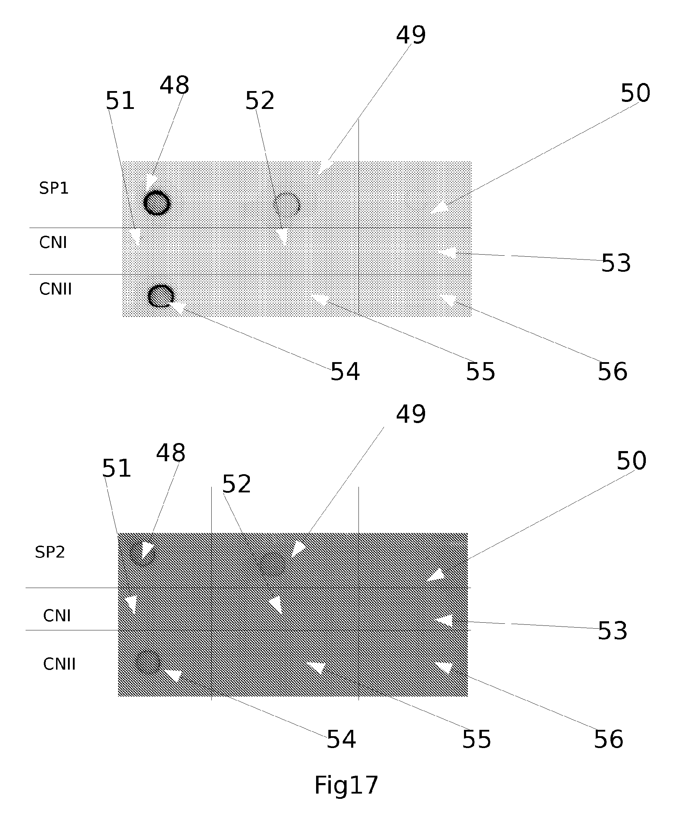

**Figure 17** **: Résultats du dotblots de la purification des molécules SP1 & SP2.**
   48 : Surnageant 1 / Molécules SP(1/2) → solution comprenant la protéines HS-LPGTG + L'enzyme sortase A + ANP SP(1/2); 49 : Élution enz AcTev/ Molécules SP(1/2) → solution comprenant la protéines HS-LPGTG + L'enzyme sortase A + ANP SP(1/2); 50 : Elution imidazole/ Molécules SP(1/2) → solution comprenant la protéines HS-LPGTG + L'enzyme sortase A + ANP SP(1/2);
   51 : Surnageant 1 /CNI → contrôle négatif 1 solution comprenant la protéines HS-LPGTG + L'enzyme sortase A ; 52 : Élution enz AcTev/CNI → contrôle négatif 1 solution comprenant la protéines HS-LPGTG + L'enzyme sortase A ; 53 : Elution imidazole/CNI → contrôle négatif 1 solution comprenant la protéines HS-LPGTG + L'enzyme sortase A ;
   54 : Surnageant 1 / CNII → Contrôle négatif II, solution comprenant la protéines HS-LPGTG + ANP SP1 ; 55 : Élution enz AcTev/ CNII → Contrôle négatif II, solution comprenant la protéines HS-LPGTG + ANP SP1; 56 : Elution imidazole/ CNII → Contrôle négatif II, solution comprenant la protéines HS-LPGTG + ANP SP1;
   Surnageant 1 : solution protéique non retenue sur les billes NI-NTA
   Elution enz Actev : 1ère élution des protéines retenues sur les billes NI-NTA par l'enzyme AcTev
   Elution imidazole : 2ème élution des protéines retenues sur les billes NI-NTA par imidazole 400mM.

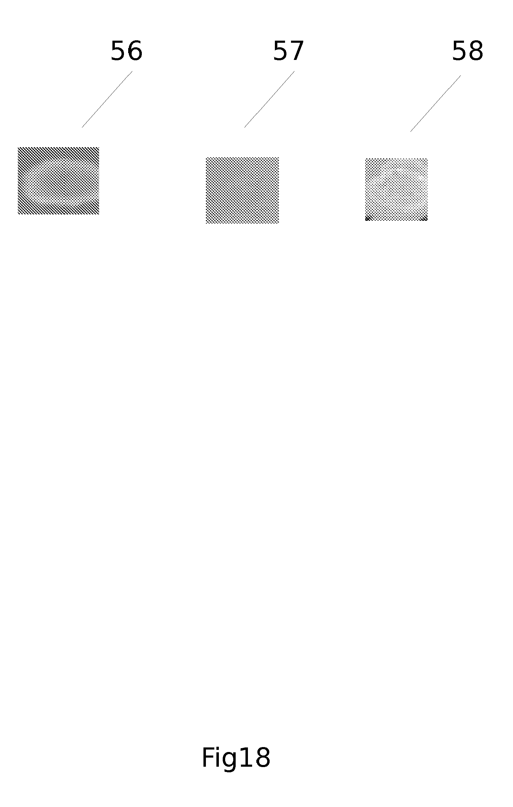

**Figure 18** **: Dotblotting Reconnaissance Antigène(HEL_DL488)/Anticorps zippé**. 56: Ac natifs anti-HEL non dilué déposés sur nitrocellulose (1x2uL à 650uM) + HEL_DL488 en solution ; 57: Ac natifs anti-HEL dilué (5*x2uL à environ 10uM) + HEL_DL488 en solution; 58 : Molécules SP1&SP2 appariées (partie variable de l'anticorps zippé)(5x2uL à environ 10uM) + HEL_DL488 en solution.

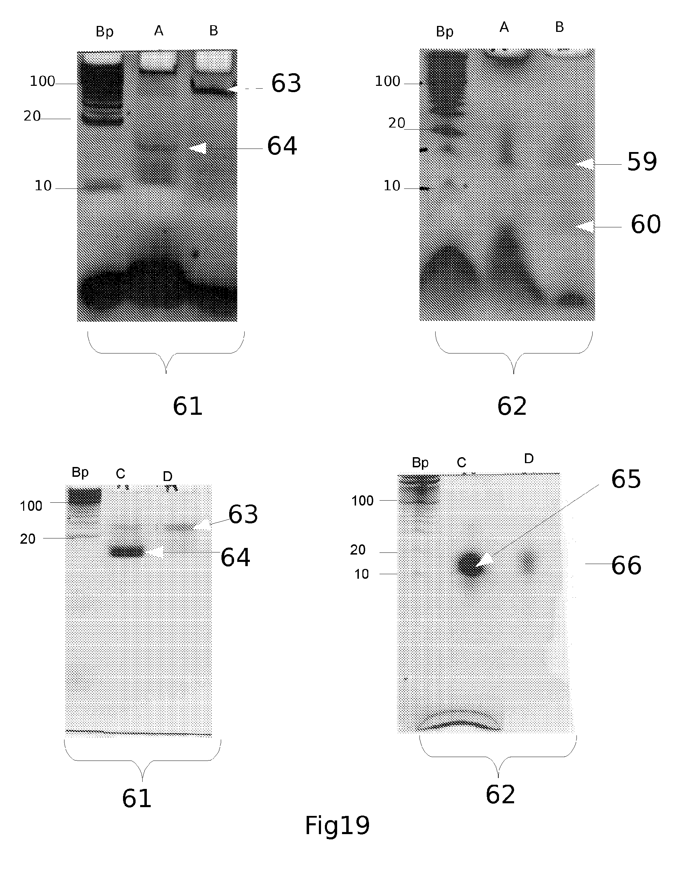

**Figure 19** **: Résultat de la digestion du PBS de HIV-I par l'ANPzyme PZ1 ;** 59 : PBS ARN non coupé ; 60 : PBS ARN coupé ; 61 : Gel natif 20% acrylamide ; 62 : Gel dénaturant 20% acrylamide ; 63 : Duplexe PBS/ANPZYME ; 64 : PBS seule ; 65 : PBS + PZ1 à T0 ; 66 PBS + PZ1 à T6h, disparition du PBS; PB : marqueur de poids moléculaires ; A : PBS (ARN) seul ; B : PBS (ARN) + PZ1 (6H à 37°C) ; C : PBS (ARN) + PZ1 (0H à 37°C) ; D : PBS (ARN) + PZ1 (6H à 37°C).

### EXEMPLES

De nouvelles molécules chimériques dites « hémi-anticorps », capables de s'auto-assembler dans un milieu approprié, sont synthétisées. Elles comprennent une partie de nature polypeptidique et une partie constituée d'une séquence nucléotidique ou d'un analogue.

Préférentiellement, la partie polypeptidique est constituée de parties d'anticorps, et plus précisément des domaines variables d'un ou plusieurs anticorps qui sont issus des chaînes lourdes et légères d'un anticorps, et qui, en se structurant forment spécifiquement un site de reconnaissance de l'épitope. La seconde partie est constituée d'un acide nucléique ou d'un analogue tel qu'un ANP ou un LNA.

Un premier type d'HAC consiste en deux domaines DV (un domaine issu d'une chaîne lourde d'un anticorps et un domaine issu d'une chaîne légère du même anticorps, qui reconnaissent spécifiquement l'antigène) et de deux séquences nucléiques.

L'un des domaines DV (figure 1.3) est couplé de façon covalente (figure 1.4) en C-terminal, soit à l'extrémité 5' d'un acide nucléique (LNA, acide nucléique méthylphosphonate, acide nucléique thioate...), soit à l'extrémité N-terminale d'un ANP.

L'autre domaine DV est couplé de façon covalente à son extrémité C-terminale, soit à l'extrémité 3' d'un acide nucléique (LNA, acide nucléique méthylphosphonate, acide nucléique thioate...), soit à l'extrémité C-terminale d'un ANP.

Les deux séquences nucléotidiques complémentaires et orientées de façon anti-parallèle s'apparient créant une hélice alpha qui permet aux deux parties protéiques d'acquérir une forme fonctionnelle. En d'autres termes, les séquences ANP et/ou acide nucléique greffées à la suite des peptides du domaine DV agissent comme une structure de type « zipper » (ANP « Zipper » ou structuraux) et les deux domaines DV se disposent en vis-a-vis pour reformer le site actif d'un hémi-anticorps (Figure 1.5a), capable de cibler un antigène intra et/ou extra cellulaire.

Cet auto-assemblage peut être utilisé pour associer, selon des assemblages nouveaux, des domaines DV nouveaux ou des domaines DV issus de domaines DV existants non associés afin de rechercher de nouvelles affinités. Cette invention décrit également un procédé de synthèse pour les chimères HAC et enfin des exemples particuliers d'utilisation de HAC spécifiques notamment pour lutter contre VIH-1 &2.

### Espaceurs/linkers peptidiques : polymère de 1 à 20 carbones :

Dans un mode de réalisation particulier, les domaines DV utilisés conservent au moins une cystéine permettant d'établir des ponts disulfures entre domaines DV. Les acides nucléiques et/ou les ANP sont alors greffés après les dites cystéines, de manière qu'en s'hybridant, les acides nucléiques et/ou ANP greffés disposent judicieusement les cystéines en permettant la formation des ponts disulfures entre les deux chaînes DV qui formeront le site actif de l'hémi-anticorps. L'appariement des séquences nucléique de type zipper permet la formation d'une molécule stable, avec un pont disulfure ayant un potentiel rédox modifié. Ce pont disulfure est maintenu en milieu intracellulaire, ce qui augmente la possibilité d'avoir un site hémi-anticorps fonctionnel.

Dans certains modes de réalisation, des molécules espaceurs linkers, telles que la Lysine, ethyl Diamine, beta alanine..., ou toute autre chaîne hydrogéno-carbonée, peuvent être intercalées entre l'acide nucléique et/ou l'ANP structural du domaine DV, de manière à ce qu'une fois les nucléotides hybridés entre eux, le décalage introduit par les espaceurs linkers entre les deux domaines DV permettent l'optimisation de la formation du site actif de l'hémi-anticorps.

### Greffage :

Selon un mode de réalisation préférentiel, un ANP (a) est greffé en C-terminal d'un domaine DV, par l'intermédiaire du NH₂ de la chaîne latérale d'une lysine liée à l'extrémité C-terminale dudit ANP (a) par une liaison peptidique. L'ANP (a) est donc d'orientation C vers N, opposée à l'orientation N vers C du DV, et la lysine joue le rôle d'espaceur linker. Le second domaine DV est liée par son extrémité C-terminale à l'extrémité N-terminale d'un autre ANP (b), grâce à une liaison peptidique ou amide avec l'un des NH₂ d'une lysine, la dite lysine étant liée, par son groupement COOH, à l'extrémité N-terminale de l'ANP (b) complémentaire de l'ANP (a). L'ANP (b) est donc orienté N vers C, de même que le DV.

Dans un mode de réalisation particulier, le greffage, d'un ANP modifié en N-ou en C-terminal par une lysine ou d'un acide nucléique modifié en 5' ou en 3' par un NH₂, sur l'extrémité C-terminale des domaines DV se fait par l'action de l'acide 2 mercapto-ethansulfonique ou par l'action de l'acide 3 mercaptopropionique sur la cystéine des DV impliquée dans la formation du pont disulfure reliant la chaîne lourde et la chaîne légère d'un anticorps.

### Domaines peptidique DV utilisés :

Les domaines DV utilisés pour réaliser les chimères peuvent provenir soit de la digestion des anticorps par la pepsine, la papaïne, la trypsine (ou toute autre enzyme), soit de l'expression des gènes codant pour ces peptides dans des systèmes cellulaires tels que des bactéries, des levures, des cellules d'insectes, ou synthèses in vitro (kits)..., puis purification. La taille de ces domaines peut varier par exemple de 10 à 300, de 20 à 200 ou de 40 à 200 résidus d'acides aminés.

### Acides nucléiques structuraux « zippers » (ou domaines nucléotidiques (ii) ; figure 1.4 ; 1.5b) :

Les acides nucléiques utilisés pour structurer l'hémi-anticorps sont des séquences complémentaires, dont la taille peut varier par exemple de 3 à 100 ou de 5 à 60 nucléotides. Ces séquences peuvent être de simples séquences nucléotidiques, des ANP, des LNA...

### Clonage :

Dans le cas du clonage des domaines DV, le domaine DV peut être fusionné à son extrémité C avec une séquence peptidique LPXTAAAA (leucine-proline-X-Thréonine-ALANINE(4) (X = un acide aminé quelconque) ; SEQ ID No. 2). L'acide nucléique ou le ANP à greffer est quant à lui fonctionnalisé à l'une de ses extrémités par un poly-G, éventuellement par l'intermédiaire d'une lysine, l'extrémité N terminale du poly-G restant libre. Le domaine DV et l'acide nucléique ou le ANP sont alors fusionnés sous l'action de sortase A.

La séquence LPXTAAAA peut être avantageusement remplacée par la séquence LPXTG (leucine - proline - X - Thréonine - Glycine, où X est un acide aminé quelconque ; SEQ ID NO. 7).

Dans un mode de réalisation particulier, il est possible de combiner des DV d'anticorps différents en les complexant grâce à des ANP structuraux complémentaires pour obtenir des nouveaux anticorps dirigés contre de nouveaux antigènes. Il est ainsi possible d'effectuer des criblages d'antigènes pour la recherche de nouveaux anticorps.

Dans un mode de réalisation particulier, les hémi-anticorps ADN/AC décrits ont à la fois la capacité d'atteindre spécifiquement la cellule ciblée (propriété de reconnaissance conférée par la partie anticorps) et celle de délivrer à l'intérieur de la cellule un domaine biologiquement actif (propriété conférée par la séquence hydrophobe additionnée). Ainsi, la molécule chimérique assure l'adressage de l'acide nucléique à la cellule mais aussi sa pénétration à l'intérieur de la cellule.

L'invention concerne également un HAC qui peut être utilisé pour cibler des cellules spécifiques afin d'y introduire par exemple un domaine nucléique biologiquement actif (ex : ANP) dirigée contre une cible particulière. Ce domaine biologiquement actif est greffé par une liaison covalente à la suite d'une des séquences nucléiques zippers décrites plus haut. Dans ce cas, le site actif ou de reconnaissance anticorps de HAC est dirigé vers des antigènes de surface. Ceci permet d'une part de reconnaître spécifiquement les cellules ciblées, et d'autre part, favorise la pénétration d'un domaine biologiquement actif en rapprochant celle-ci de la membrane.

En d'autres termes, la molécule chimérique est constituée des parties variables d'un hémi-anticorps DV1 a/DV1 b (figure 1.5a). Sur chaque peptide DV est greffé un acide nucléique structural (zipper), de telle sorte que cet acide nucléique greffé sur un peptide DV1 a est complémentaire et antiparallèle à l'acide nucléique structural (zipper) greffé sur le deuxième peptideDV1b. Les deux peptides DV forment alors un hémi-anticorps avec son site actif ou de reconnaissance. Un des deux acides nucléiques structuraux se continue par une séquence nucléotidique (tag) (figure 1.6 à 1.8) comportant un domaine biologiquement actif.

Dans un mode encore plus particulier l'hémi-anticorps permet de cibler des organelles ou des molécules intracellulaires.

### Domaine biologiquement actif (ou domaine Tag ; figure 1.6 à 1.9)

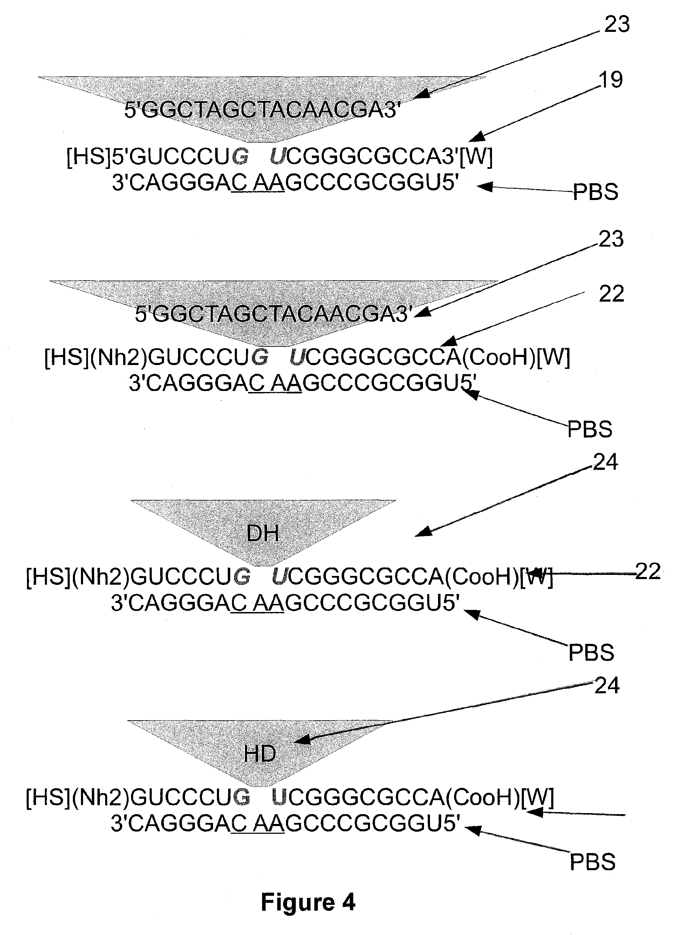

Le domaine biologiquement actif peut être un acide nucléique, un ANP, une séquence mixte ANP/acide nucléique, ANP/peptide et plus généralement une séquence nucléotidique ou un analogue (ANP, LNA, méthylphosphonates, méthylsulfonates, acides nucléiques ou desoxy nucléiques modifiés ou un mélange de ces différentes molécules...) une ADNzyme, une ANPzyme, ou un MiRNA, éventuellement un antisens, qui en entrant dans la cellule cible empêchera l'expression d'une séquence cible ou la dégradera ou présentera une activité DNAase ou RNAase , ou une combinaison de ces deux propriétés (figure 4.19).

Dans un mode de réalisation particulier le domaine biologiquement actif est une séquence antisens comprenant tout ou partie de L'ARNt^{Lys3}, par exemple GTCCC ou GUCCC, complémentaire d'une partie de l'ARN viral VIH-1.

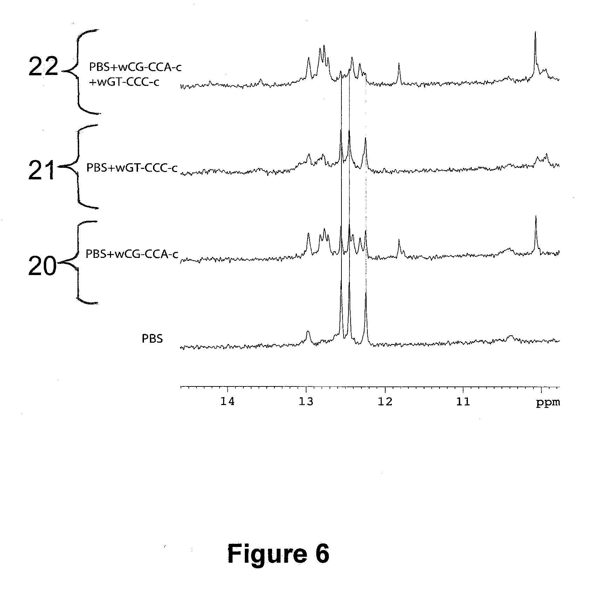

Dans la figure 6.21, est présentée l'interaction, mise en évidence par RMN, entre un ANP w-GTCCC-c (w tryptophane et c cystéine avec N terminal 5' et C terminal 3') et les séquences Primer Binding Site (PBS) de VIH-1. La séquence forme un complexe extrêmement stable avec le PBS de VIH-1, capable de déplacer le complexe ARNt^{ARNtLys3}/ PBS de VIH-1.

Dans un autre mode de réalisation le domaine biologiquement actif est une séquence antisens comprenant tout ou partie de la séquence terminale du tRNA_lys3 par exemple CGCCA, complémentaire d'une partie de l'ARN viral VIH-1.

Dans la figure 6.20 est présentée l'interaction, mise en évidence par RMN, entre un ANP w-CGCCA-c (W tryptophane et c cystéine avec N terminal 5' et C terminal 3') et les séquences PBS de VIH-1. La séquence forme un complexe extrêmement stable avec PBS de VIH-1, capable de déplacer le complexe ARNtARNtLys3/ PBS VIH-1.

Dans un mode de réalisation encore plus particulier le domaine biologiquement actif proprement dit est une combinaison des deux séquences GTCCC ou GUCCC et CGCCA (figure 6.22) (N' ATGGTAGAG-cystéine C' & N' cystéine-CTCTACCAT C').

Dans un mode de réalisation particulier, la séquence antisens proprement dite est une séquence complémentaire à tout ou partie de la région PBS ou de la région TAR de l'ARN viral de VIH-1 ou VIH-2.

Dans un mode de réalisation particulier, le domaine biologiquement actif antisens comprend des ANP (figure 6.22).

Dans un mode de réalisation particulier, le domaine biologiquement actif est une séquence à activité enzymatique de type DNAzyme et des séquences de reconnaissance complémentaires de tout ou partie de la séquence PBS.

Dans un mode de réalisation encore plus particulier, le domaine biologiquement actif est constitué d'un dimère DNA/ANP-zyme. La partie ANP (flanquant de part et d'autre la boucle DNA-Zyme) est complémentaire d'une séquence cible d'intérêt qui peut être tout ou partie de la séquence PBS ou TAR du VIH-1 ou VIH-2 (figure 4.22) et la partie DNA-zyme, formée de 2 à 23 résidus, présente une structure en boucle (Figure 4.23) et entraînera la dégradation de la séquence cible de par son activité RNase ou DNase.

Dans un mode de réalisation particulier, la totalité de la séquence enzymatique est un ANP, formant ainsi un ANP-zyme.

Dans un mode de réalisation particulier, la boucle enzymatique de l'ANPzyme est complètement ou partiellement substituée par un motif comprenant au moins un acide aminé tel que DH (acide aspartique - histidine), HD (histidine - acide aspartique), EH (acide glutamique - histidine) ou HE (histidine - acide glutamique) (figure 4.24).

Dans un mode de réalisation particulier, la séquence ANPzyme, antisens, ANP/DNAzyme proprement dite est utilisée seule ou en combinaison de n'importe quelle partie de l'HAC_Tag (HAC, séquence espaceur, extrémité lipophile ou aromatique).

Le système de séquence biologiquement active peut être adapté à tous types de virus, de bactéries notamment en ciblant les gènes de résistance aux antibiotiques afin d'inhiber la résistance et de rendre les bactéries sensibles à l'antibiotique correspondant.

Dans un mode de réalisation particulier, la séquence anti-sens proprement dite comprend tout ou partie de la séquence ARN ou ADN des gènes bactériens de résistance aux antibiotiques ou une séquence anti-sens complémentaire à tout ou partie de cette séquence.

### Espaceurs/linkers nucléiques (figure 1.6) :

La séquence greffée peut comporter une séquence espaceur de 5 à 50 nucléotides visant à optimiser la distance entre le domaine nucléique ii) et le domaine biologiquement actif qui doit pénétrer dans la cellule.

Dans un mode de réalisation préférentiel, la séquence tag comprend une partie acide désoxyribonucléique faisant office d'espaceur (figure 1.6). L'espaceur est relié à une séquence ANP (ou à un autre acide nucléique, modifié ou non) constituant le domaine biologiquement actif proprement dit (figure 1.8).

Le domaine biologiquement actif est par exemple relié par une de ses extrémités à la séquence espaceur par une séquence labile en milieu réducteur par exemple un pont disulfure SS (figure 1.7).

Dans un mode de réalisation particulier, la séquence espaceur est absente et le domaine biologiquement actif est relié à l'hémi-anticorps par une liaison covalente.

Dans un mode de réalisation particulier, une fonction thiol est présente à l'autre extrémité libre de la séquence espaceur. Cette fonction permet de greffer une autre molécule sur la séquence espaceur par l'établissement, par exemple, d'une liaison SS. Cette liaison SS est labile en milieu réducteur tel que le milieu du cytoplasme cellulaire. Cette liaison, après réduction du pont SS, permet la séparation du HAC et du domaine biologiquement actif. Selon un mode de réalisation particulier de l'invention, la fonction thiol est introduite à l'extrémité de la séquence espaceur par fusion, avec la dite séquence, d'une séquence comportant une fonction thiol à une des extrémités. La fusion de la séquence espaceur et de la séquence comportant une fonction thiol à son extrémité libre peut être obtenue grâce à une ligase.

Le domaine biologiquement actif peut, par exemple, comporter une fonction thiol à une de ses extrémités pour l'établissement d'un pont disulfure entre le domaine biologiquement actif et la séquence espaceur. Cette fonction thiol peut être remplacée par un acide maléïmide afin que la liaison entre la séquence espaceur et le domaine biologiquement actif soit persistante en milieu réducteur.

### Passage transmembranaire:

La cible de cet HAC peut-être extra ou intracellulaire, ce qui implique, dans ce dernier cas, le passage de l'acide nucléique à travers la membrane plasmique. Ce passage est rendu possible par l'addition d'une séquence hydrophobe à l'extrémité libre de l'acide nucléique (domaine biologiquement actif) à transférer. Ce groupement lipophile (figure 1.9) permet au domaine biologiquement actif de traverser la membrane cellulaire plus facilement. Dans un mode de réalisation encore plus préférentiel, le groupement lipophile est aromatique de manière à s'intercaler dans une hélice mixte ANP/Acide nucléique afin de stabiliser cette structure formée entre l'antisens ANP et la cible acide nucléique.

Le site de reconnaissance de la structure HAC_tag se fixe spécifiquement sur les cellules comportant les antigènes cibles ; la fonction lipophile greffée sur le domaine biologiquement actif (ANP antisens, DNAzyme...) permet au bras nucléotidique de traverser la membrane, et plus particulièrement, au domaine biologiquement actif. Le milieu intracellulaire étant un milieu réducteur, les ponts SS reliant le domaine biologiquement actif à la séquence espaceur sont réduits, libérant ainsi le domaine biologiquement actif à l'intérieur de la cellule cible.

Par exemple l'ANP antisens se complexer alors avec les séquences sens cibles (ARN dans le cytoplasme ou ADN dans le noyau) et bloque les possibilités d'expression de la séquence cible (au niveau de la transcription, rétrotranscription, réplication, et/ou traduction). La molécule aromatique, telle que le tryptophane introduit à l'extrémité du ANP antisens, stabilise le duplex ANP/ADN ou ARN qui se forme, et améliore ainsi l'efficacité de blocage par l'ANP.

### Anticorps ou domaines peptidique DV

Dans un mode de réalisation particulier, l'anticorps utilisé pour réaliser le HAC_tag est un anticorps reconnaissant une protéine d'une enveloppe virale ou bactérienne.

Dans un mode de réalisation particulier, l'anticorps utilisé pour réaliser l'HAC_tag est capable de se fixer sur la particule virale.

Dans un mode de réalisation dédié, l'anticorps utilisé pour réaliser l'HAC_tag est l'anticorps anti gp120-17b. L'HAC_tag-gp120-17b peut se fixer sur les particules virales et est conduit par elles jusqu'aux cellules cibles du virus grâce aux interactions CD4/gp120. Après fixation des virions sur les cibles, le bras nucléique de l'HAC-tag peut traverser la membrane cellulaire grâce à la fonction lipophile greffée à l'extrémité du domaine biologiquement actif. Après passage au travers de la membrane cellulaire, le pont SS, reliant la séquence espaceur et le domaine biologiquement actif, est réduit dans le cytosol, libérant le domaine biologiquement actif dans la cellule. La séquence biologiquement active bloque ou bloque et dégrade la séquence virale, selon que domaine biologiquement actif est un antisens ou un antisens/ANPzyme.

Selon un mode de réalisation particulier, l'anticorps utilisé pour la synthèse de l'hémi-anticorps est constitué d'un anticorps anti-retrotranscriptase notamment anti-retrotranscriptase VIH-1 ou VIH-2. Dans cette configuration, l'hémi-anticorps est relié au domaine biologiquement actf par l'intermédiaire d'une liaison persistante en milieu réducteur, par exemple par l'intermédiaire d'un acide maléïmide. Une séquence espaceur peut éventuellement être intercalée entre l'hémi-anticorps et le domaine biologiquement actif, les liaisons reliant HAC, séquence espaceur et domaine biologiquement actif étant persistantes en milieu réducteur. Le domaine biologiquement actif est préférentiellement une séquence ANPzyme, DNAzyme ou ANP antisens. Le HAC-tag ainsi constitué se complexe à la reverse transcriptase alors que le domaine biologiquement actif dégrade et/ou bloque l'ARN viral.

Les HAC_tag peuvent être inclus (en particulier encapsidé) dans des microsphères ou des liposomes, plus particulièrement les HAC_tag seront inclus dans des liposomes constitués de membrane virale.

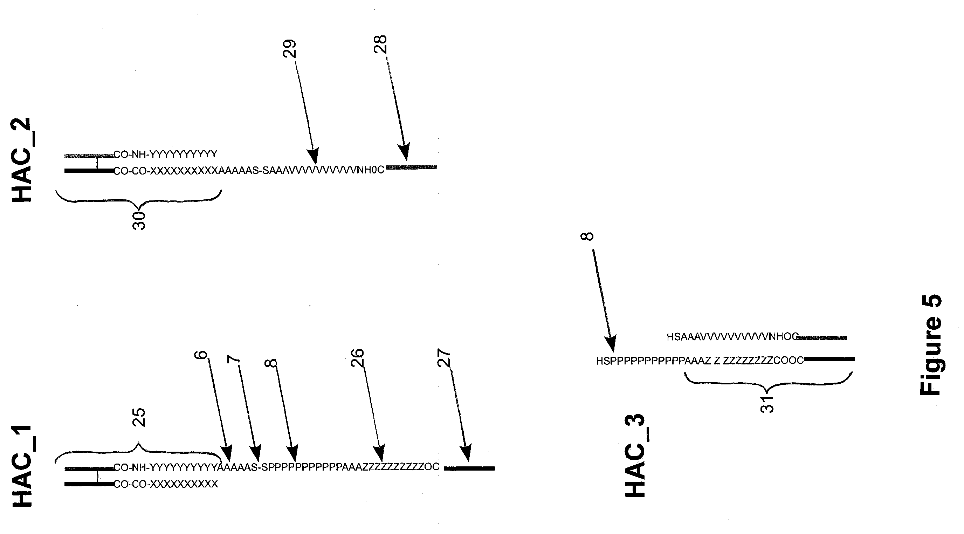

L'invention concerne également un système comprenant (voir figure 5):
1) un premier HAC (HAC_1 ; figure 5.25), dirigé par exemple contre un antigène présent à la surface des cellules ou un antigène viral et comprenant ou constitué de:
   ∘ deux domaines DV (DV1 a et DV1 b), liés respectivement à
   ∘ deux séquences zippers (S1 et S2) complémentaires, antiparallèles
   ∘ un espaceur lié à une séquence zipper de HAC_1, lié par une de ses extrémités et par l'intermédiaire d'un pont SS à,
   ∘ un domaine biologiquement actif (figure 5.8), lié par le biais d'une liaison covalente à
   ∘ un acide nucléique structural, S3 (figure 5.26), d'un troisième HAC (HAC_3),
   ∘ l'autre extrémité de la séquence structurale (S3) étant liée à
   ∘ un domaine DV (DV2a ; figure 5.27);
2) un second HAC (HAC_2), dirigé par exemple contre un antigène présent également à la surface des cellules et comprenant ou constitué de:
   ∘ deux domaines DV (DV3a et DV3b), liés respectivement à
   ∘ deux séquences zippers (S5 et S6) complémentaires, antiparallèles
   ∘ un espaceur comportant un pont disulfure ou lié par l'intermédiaire d'un pont disulfure à,
   ∘ un acide nucléique structural, S4 (figure 5.29), complémentaire, antiparallèle à S3, lié à
   ∘ un domaine DV (DV2b ; figure 5.28).
3) Dans une cellule comportant HAC_1 et HAC_2, un troisième HAC, HAC_3 se forme. Celui-ci comprend :
   ∘ deux DV (DV2a et DV2b), liés aux
   ∘ deux acides nucléiques structuraux, S3 et S4, complémentaires, antiparallèles (dérivés respectivement de HAC_1 et HAC_2),
   ∘ S3 étant relié par le biais d'une liaison covalente au
   ∘ domaine biologiquement actif dérivé de HAC_1 (figure 5.8).

Selon un mode de réalisation particulier HAC_1 est dirigé contre la protéine gp120, HAC_2 est dirigé contre la protéine CD4.

Selon un mode de réalisation particulier, DV2a et DV2b forment un site de reconnaissance HAC_3 (figure 5.31) anti retro-transcriptase lorsque les deux acides nucléiques structuraux S3 et S4 s'hybrident.

Ce système peut être décliné de diverses manières pour délivrer, à l'intérieur d'une cellule, les deux parties d'un hémi-anticorps, qui peuvent ainsi s'auto-assembler dans la cellule. On peut notamment utiliser des particules virales pour délivrer spécifiquement les hémi-anticorps et leur domaine biologiquement actif dans la cellule.

### Préparation des molécules chimériques par fusion de ponts disulfures.

### a) Réacteur

L'anticorps subit une réduction électrochimique ménagée dans un réacteur refroidi. Le réacteur se compose d'un tube vertical de laiton de 2 mm de diamètre et de 1,5 cm de longueur contenant la solution d'anticorps à électroréduire. L'extrémité inférieure du tube est reliée à la borne positive d'un générateur par une électrode mixte composée d'une aiguille remplie et entourée d'un de gel duracryl 20 % (124 µL de duracryl (*Sigma*) 30% + 30 µL de Tris 1,5M pH 8,9 + 43,6 µL H₂O + 50 µL de Tampon d'électroréduction + 5 µL APS 10% + 2 µL de Temed) contenu dans un tube Tycon (N*orton*) de 1.5 cm, fermé d'un côté à l'aide de la flamme d'un bec Bunsen. L'électrode gel permet le passage des électrolytes, mais limite la diffusion des anticorps ou de leurs formes réduites. L'extrémité supérieure du réacteur est reliée au pôle négatif du générateur par une aiguille, de manière à empêcher tout contact entre les parois du réacteur et l'électrode grâce à un cône de pipette qui guide l'aiguille à l'intérieur du réacteur. Le réacteur est entouré par un système de refroidissement constitué d'un autre tube en laiton torsadé pour former une spirale dans lequel circule de l'eau à la température de la glace fondante, sous à l'action d'une pompe péristaltique. Les paramètres utilisés pour la réduction de l'anticorps en hémi-anticorps sont : +100V, 2mA, 10 sec.

### b) Fonctionnalisation des hémi-anticorps par oxydation des fonctions thiols.

Une solution de 4 µL d'anticorps anti-BSA à 0,5 µg/µL, 4 µL de tampon 0,12% β-mercaptoéthanol et de 6 µL de la séquence Tag_C à 500 ng/µL (^{5'}HS-TCAAGTAGCTTCAGAATCTTTTCCCCCC^{3'}; SEQ ID No. 3), est utilisée pour fonctionaliser les fonctions Thiols (SH) libérées sur les anticorps après réduction par électro-oxydation à -100V, 2mA, pendant 10 secondes.

### Mise en évidence par fluorescence

Le marquage des séquences Tag_C des chimères est réalisé grâce à une séquence nucléotidique (tag_D = ^{5'}CTGAAGCT³_cystéine) complémentaire de la séquance tag_C et fonctionnalisée en 3' par un fluorophore Cyanine 3. 15 µL de séquence tag_D (0,3 µg/µL) sont ajoutés à 10 µL d'une solution de chimères fonctionnalisées par une séquence tag_C et diluée (1/1000) dans du lait/TBS1X. Dans cette solution, un dépôt d'antigène BSA sur membrane nitrocellulose est incubé toute la nuit à 4°C. Après trois lavages avec du TBS 1X pendant 5 minutes pour éliminer les accrochages a spécifiques, la membrane est dénaturée par 50 µL de β-mercaptoéthanol 2,5%, à une température de 95°C, pendant 20 minutes. 2 µL de surnageant sont déposés sur une lame de verre. Le dépôt est séché 20 minutes à l'air libre et à l'abri de la lumière, puis la lame est scannée (*Packard Bioscience*) à 455 nm.

Un contrôle négatif est effectué en parallèle : une membrane comportant un dépôt d'antigène est incubée avec 15 µL de tag_D et 1 mL de lait/TBS 1X à 4°C toute la nuit avec agitation. La membrane subit le même protocole que précédemment. 2 µL provenant de sa dénaturation sont de même déposés sur lame de verre et scannés pour évaluer l'accrochage aspécifique.

### c) fusion des domaine (i) DV et (ii) Zipper par un protocole de transpeptidation enzymatique par Sortase A (Fig-16)

### c.1) Sortase A

Le plasmide pLLC092 (http://isisbiolab.dabbledb.com/publish/laboratorvstocks/ff0e3c28-ca9a-4280-95ce-f170e7f7a39a/allentries.txt) contenant la séquence de la sortase contiguë à un Tag Histidine, a été transfecté dans les cellules compétentes BL21(DE3) (biolabs) sous le contrôle d'un promoteur Lac induit par l'IPTG (isopropyl β-D-1-thiogalactopyranoside). L'expression de la sortase A est alors induite par IPTG (1 mM). L'enzyme sortase A est ensuite purifiée par le kit Ni-NTA Fast Start Kit (Qiagen).

### c.2) Protéines HS-LPGTG & LS-LPGTG

Les deux séquences (I-A) HS-LPGTG et (I-B) LS-LPGTG ci-dessus correspondant respectivement à la partie variable de la chaîne lourde et de la chaîne légère de l'anticorps anti-HEL (Hen Egg Lysozyme) ont été clonées dans le vecteur pEXP5-NT (invitrogen) par Proteigenix. Les vecteurs ont été transfectés séparement dans des bactéries compétentes Top10 one shot E.coli (invitrogen). La purification des plasmides a été faite avec le kit QIAfilter Plasmid Midi Kit (Qiagen). L'expression des deux protéines HS-LPGTG & LS-LPGTG a été réalisée grâce au Kit expressway cell-free E.coli expression system (invitrogen). Les deux protéines synthétisées ont ensuite été purifiées par le kit Ni-NTA Fast Start Kit (Qiagen).

### Deux ANP Zipper (domaine ii-A) SP1 & (ii-B) SP2 de séquence respective

ATGGTAGAG et CTCTACCAT prolongés par les linkers N'GlyGlyGly(CO)NH(CH2)4(NH2)CH(COO) et NH2-CH(CONH2-(CH2)4NH(CO)GlyGlyGly N'), ont été synthétisées de manière à obtenir
SP1 (N'GlyGlyGly(CO)NH(CH2)4(NH2)CH(CO)NHATGGTAGAGTTTTTC') et
SP2 (N' CTCTACCAT-CONH-CH(CONH2)-(CH2)4NH(CO)GlyGlyGly N').

Une séquence TTTTTC est ajouté en fin de SP1 afin de pouvoir mettre en évidence la chimère formée en la marquant par un acide nucléique GAAAAA fluorescent.

SP1 et SP2 sont repris dans une solution de 10% N-méthyl-2-pyrrolidone (NMP) pour concentration finale de 100 micro M.

Test-I : Une solution de 50 uL de SP1 et une solution de 50 uL de SP2, sont préparées à une concentration finale de 20uM contenant 20uM de HS-LPGTG (I-A), et respectivement 20uM LS-LPGTG (I-B), l'enzyme SrtA à 20uM, Tris-HCL pH 7.5 à 50mM , NaCl à 150mM et CaCl2 à 5mM.
quatre contrôles négatifs sont également réalisés (Fig-17) :
- CNI-sp1 (solution sans ANP) 50uL de solution contenant HS-LPGTG (I-A) à 20uM et l'enzyme SrtA à 20uM.
- CNII-sp1 (solution sans srtA) 50uL de solution contenant HS-LPGTG (I-A) à 20uM et le ANP-SP1 à 20uM.
- CNI-Sp2 (solution sans ANP) 50uL de solution contenant LS-LPGTG (I-B) à 20uM et l'enzyme SrtA à 20uM.
- CNII-SP2 (solution sans srtA) 50uL de solution contenant LS-LPGTG (I-B) à 20uM SP2 à 20uM.

Les tubes sont incubés 24H à 37°C.

### c.3 Purification par billes magnétiques et enzyme AcTev

25uL de billes Ni-NTA magnetic agarose beads (Qiagen) sont précipitées, le surnageant est enlevé. Ajout sur ces billes des 50uL de solution à purifier + 100uL de protein binding buffer (50mM NaH2PO4, 300 mM NaCl, 20 mM imidazole, pH8). Incubation 1H30 sur roue à température ambiante à 7rpm. Précipitation des billes récupération du surnageant n°1. Deux lavages des billes avec 150 uL de protein binding buffer. Coupure de l'étiquette 6histidine par l'enzyme AcTev protéase (Invitrogen) 10uL de 5X Tev Buffer, 5uL de 0.01 M de DTT, 0.5uL d'enzyme AcTev protéase, 34.5uL dH2O, incubation 4H à 30°C. Précipitation des billes, récupération du surnageant n°2 (contenant les molécules ayant subi la transpeptidation). Deux lavages des billes avec 150 uL de protein binding buffer. Elution des protéines encore présentes sur billes par 50uL d'Elution protein buffer (50mM NaH2PO4, 300 mM NaCl, 250 mM imidazole, pH8) pendant 1 H à température ambiante sur roue à 7rpm. Précipitation des billes récupération du surnageant n°3.

### c.4 Mise en évidence de la fusion (cf Fig-17).

Pour SP1 et respectivement SP2, 2uL de chaque surnageant (test, CNI, CNII) récupérés sont déposés sur une membrane de nitrocellulose protean (Whatman). Séchage des dépôts 20 minutes à température ambiante. Saturation avec une solution de lait 5%/TBS1X pH7.6 à 4°C pendant 20minutes. Hybridation de la membrane toute la nuit à 4°C sous légère agitation dans un tampon de lait 5%/TBS1X pH7.6 contenant 50pM de séquences, marquées par fluorescence en Cy-3 et 6-FAM respectivement, complémentaires au ANP-SP1 ou au ANP-SP2 respectivement. Trois lavages de 15 minutes de la membrane avec une solution de lait 5%/TBS1X pH7.6. Scan au Typhoon 8600 (Amersham Biosciences).

Les résultat sont présenté, démontre la fusion entre la molécule SP1 et SP2 avec les peptides HS-LPGTG et LS-LPGTG respectivement pour obtenir SP1(I-II) et SP2(I-II).

### d) Auto assemblage de la chimère.

### d.1) Hybridation des molécules SP1 (i-ii) avec les molécules SP2 (I-II)

12,5uL de surnageant N°2 produit en C.3 (cf ci-dessus) de molécules SP1(I-II) et 12,5uL de surnageant N°2 produit en C.3 (cf ci-dessus) de molécules SP2 (I-II) + 25uL de tampon neutre (200uL de glycérol 99%, 33uL de tris 3M pH6.8, 267uL dH2O) sont mis à hybrider 1 H à 37°C.

### d.2) Vérification de la fonctionnalité de la molécule (cf-fig 18).

5 fois 2 µL de solution obtenue en D.1 sont déposés sur une membrane de nitrocellulose protean (Whatman) avec un séchage de 10 min entre chaque dépôt de manière a former une zone de dépôt ou spot.

Deux contrôles sont effectués tels que :
a) 2ul d'anticorps anti-HEL (Tebu-bio) à 0,1ug/uL et b) 5 fois 2 µL d'anticorps anti-HEL (Tebu-bio) à 10 uM (concentration comparable à la molécule appariée) sont respectivement déposés sur la même membrane avec un séchage de 10 min entre chaque dépôt à température ambiante de manière à former respectivement deux autre zones de dépôts.

La membrane de nitrocellulose est alors saturée par une solution de superblocking buffer (Thermofisher) à 4°C pendant 20minutes, puis hybridées, toute la nuit à 4°C sous légère agitation, avec une solution contenant 250nM d'antigène HEL (Fluka) marqué Dylight488 (Thermofisher) dans du superblocking buffer. Trois lavages de 15 minutes de la membrane avec du superblocking buffer. Scan au Typhoon 8600 (Amersham Biosciences).

Les résultats figure(Fig-18) démontrent que la protéine chimérique capture son antigène et est donc fonctionnelle après l'étape d'auto assemblage par hybridation.

### e) Exemple d'ANPZYME: Coupure du PBS (HIV-1) cf (Fig-19)

Une solution d'ARN « Primer Binding Sequence » (ou PBS) du HIV-1 de 18 bases (5' UGGCGCCCGAACAGGGAC 3' ; SEQ ID NO.10) à 10uM dans H20 est mélangé mole à mole, soit à une solution de ANPzyme (N' TCCCTGHDTC C') à 10uM dans du NMP 10%, soit à une solution de NMP 10%. Les mélanges sont incubés 4H à 37°C. Les mélanges sont déposés soit dans un gel natif à 20% d'acrylamide (1.5mL de TBE10X, 7.5mL d'acrylamide/bis 19:1 (biorad), 6mL dH2O, 87.5 uL d'APS 10% et 7.5 uL de temed (Sigma)) ; soit dans un gel dénaturant à 20% d'acrylamide (7.2g d'urée (Sigma), 1.5mL de TBE10X, 7.5 d'acrylamide/bis 19:1 (Biorad), 6mL dH2O, 87.5 uL d'APS 10% et 7.5 uL de temed (Sigma)). Après Migration à 100V et 200mA pendant 1h30, les gels sont révélés par du Bromure d'éthidium.

L'analyse des gels montre que l'ANPZYME s'hybride avec le PBS et est capable de couper la molécule de PBS.

### REFERENCES

David Baulcombe. 2002. An RNA Microcosm. Science, 297: 2002-2003.
Rong Fan, Ophir Vermesh, Alok Srivastava, Brian K H Yen, Lidong Qin, Habib Ahmad, Gabriel A Kwong, Chao-Chao Liu, Juliane Gould, Leroy Hood & James R Heath. 2008. Integrated barcode chips for rapid, multiplexed analysis of proteins in microliter quantities of blood. Nature Biotechnology. Vol. 26, No. 12: 1373 - 1378.
Muthiah Manoharan, Kathleen L. Tivel and P. Dan Cook. 1995. Lipidic nucleic acids. Tetrahedron Letters. Vol. 36, No. 21: 3651-3654.
Indriati Pfeiffer and Fredrik Höök. 2004. Bivalent Cholesterol-Based Coupling of Oligonucletides to Lipid Membrane Assemblies. J. Am. Chem. Soc. 126 (33): 10224-10225.
Snehlata Tripathi, Binay Chaubey, Sabyasachi Ganguly, Dylan Harris, Ralph A Casale, and Virendra N. Pandey. 2005. Anti-VIH-1 activity of anti-TAR polyamide nucleic acid conjugated with various membrane transducing peptides. Nucl. Acids Res. Vol. 33, No. 13: 4345-4356.

## Revendications

1. Molécule chimérique appelée « hémi-anticorps », **caractérisée en ce qu'**elle comprend ou consiste en deux molécules chimériques A et B comprenant ou consistant chacune en :
(i) un domaine polypeptidique caractéristique d'un domaine variable (ou DV) d'une chaîne lourde ou d'une chaîne légère d'un anticorps, ce domaine polypeptidique étant positionné à une extrémité dans les molécules chimériques A et B, le domaine polypeptidique (i) d'une des deux molécules chimériques, A ou B, étant caractéristique d'un DV d'une chaîne légère d'un anticorps et le domaine polypeptidique (i) de l'autre molécule chimérique, respectivement B ou A, étant caractéristique d'un DV d'une chaîne lourde d'un anticorps; et
(ii) un domaine nucléotidique simple brin consistant en un polynucléotide, ou un analogue d'un polynucléotide, le domaine nucléotidique de A et celui de B étant capables de s'apparier en une structure double-brin en milieu hydrique;
le domaine polypeptidique (i) et le domaine nucléotidique (ii) de chacune des molécules chimériques A et B étant liés par une liaison covalente, et le domaine (ii) de la molécule chimérique A et de celui de la molécule chimérique B étant appariés en une structure double brin en milieu hydrique.

2. Hémi-anticorps selon la revendication 1, dans lequel le domaine polypeptidique (i) et le domaine nucléotidique (ii) d'une molécule chimérique A ou B sont liés par une ou plusieurs molécule(s) de liaison (ou linker(s)), ledit ou lesdits linker(s) comprenant ou consistant en une chaîne hydrogéno-carbonée, et comprenant ou consistant en un ou plusieurs élément(s) choisi(s) parmi un peptide ou un polypeptide, un polynucléotide ou un analogue d'un polynucléotide, une chaîne polycarbonnée, ethylènediamine, polylysine, bêta-alanine, et un sucre.

3. Hémi-anticorps selon la revendication 1 ou 2, dans lequel:
- dans la molécule chimérique A, l'extrémité C-terminale du domaine polypeptidique (i) est liée de façon covalente à l'extrémité 3' d'un acide nucléique ou à l'extrémité C-terminale d'un analogue d'un polynucléotide constitué par un acide nucléique peptidique (ANP); et/ou
- dans la molécule chimérique B, l'extrémité C-terminale du domaine polypeptidique (i) est liée de façon covalente à l'extrémité 5' de l'acide nucléique ou à l'extrémité N-terminale d'un analogue d'un polynucléotide constitué par un acide nucléique peptidique (ANP).

4. Hémi-anticorps selon la revendication 3, dans lequel :
- dans la molécule chimérique A, la liaison covalente est établie par l'intermédiaire du groupement NH₂ de la chaîne latérale d'une lysine liée à l'extrémité C terminale dudit ANP par une liaison peptidique ; et/ou
- dans la molécule chimérique B, la liaison covalente est établie par l'intermédiaire d'une liaison peptidique ou amide avec l'un des groupements NH₂ d'une lysine, ladite lysine étant liée, par son groupement COOH, à l'extrémité N-terminale dudit ANP.

5. Hémi-anticorps selon l'une quelconque des revendications 1 à 4, dans lequel la molécule chimérique A et/ou la molécule chimérique B comprend (comprennent) en outre un ou plusieurs élément(s) choisi(s) parmi :
• un domaine étiquette et/ou un domaine biologiquement actif, qui est un polynucléotide ou un analogue comprenant ou consistant en une séquence étiquette (ou séquence TAG) et/ou une séquence biologiquement active permettant de bloquer l'expression de gènes cibles, ledit polynucléotide ou ledit analogue étant choisi parmi les éléments suivants:
- un acide nucléique, un acide nucléique antisens ou un acide nucléique ayant une activité endonucléase;
- un ANP, un ANP antisens ou un ANP ayant une activité endonucléase (ANPzyme),
- un LNA, un acide nucléique méthylphosphonate, un acide nucléique méthylsulfonate, un acide nucléique ou desoxy-nucléique modifié, une ADNzyme, une ARNzyme ou une ANPzyme, un micro ARN (miRNA), une séquence mixte ANP/acide nucléique ou ANP/peptide, un dimère DNA/ANP-zyme ; et
- une combinaison d'au moins deux de ces éléments,
ledit domaine étiquette ou le domaine biologiquement actif étant lié à l'extrémité libre du domaine nucléotidique (ii) de la molécule chimérique A ou B ou à l'extrémité libre d'un linker lié au domaine nucléotidique (ii) de la molécule chimérique A ou B, ledit linker étant tel que défini à la revendication 2, et la liaison dudit domaine étiquette ou dudit domaine biologiquement actif s'effectuant par l'intermédiaire d'une liaison labile en milieu réducteur, ou par l'intermédiaire d'une liaison persistante en milieu réducteur; et
• un élément lipophile choisi parmi une séquence, un groupement chimique ou un composé chimique lipophile, qui comporte un ou plusieurs groupement(s) et/ou composé(s) aromatique(s), ledit élément lipophile étant positionné à l'extrémité libre de la molécule chimérique A ou B.

6. Hémi-anticorps selon l'une quelconque des revendications 1 à 5, dans lequel :
• la première molécule chimérique, A ou B, comprend ou consiste en:
- un domaine polypeptidique (i) tel que défini à la revendication 1, et les domaines suivants, positionnés successivement dans l'ordre suivant, à partir de l'extrémité C-terminale du domaine polypeptidique (i):
- un linker tel que défini à la revendication 2,
- un domaine nucléotidique (ii) tel que défini à la revendication 1,
- un linker tel que défini à la revendication 2, lié par l'intermédiaire d'une liaison labile en milieu réducteur ou par l'intermédiaire d'une liaison persistante en milieu réducteur, à
- un domaine étiquette ou un domaine biologiquement actif tel que défini à la revendication 5, et
- un élément lipophile tel que défini à la revendication 5; et
• la deuxième molécule chimérique, respectivement B ou A, comprend ou consiste en :
- un domaine polypeptidique (i) tel que défini à la revendication 1, ce domaine et le domaine polypeptidique (i) de la première molécule chimérique étant caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
et les domaines suivants, positionnés successivement dans l'ordre suivant, à partir de l'extrémité C-terminale du domaine polypeptidique (i):
- un linker tel que défini à la revendication 2, et
- un domaine nucléotidique (ii) tel que défini à la revendication 1.

7. Hémi-anticorps selon l'une quelconque des revendications 1 à 6, dans lequel :
• la première molécule chimérique, A ou B, comprend ou consiste en:
- un domaine polypeptidique (i) tel que défini à la revendication 1, et les domaines suivants, positionnés successivement dans l'ordre suivant, à partir de l'extrémité C-terminale du domaine polypeptidique (i):
- un linker tel que défini à la revendication 2,
- un domaine nucléotidique (ii) tel que défini à la revendication 1, appelé S1
- un linker tel que défini à la revendication 2, lié par l'intermédiaire d'une liaison labile en milieu réducteur à
- un domaine étiquette ou un domaine biologiquement actif tel que défini à la revendication 5, lié par l'intermédiaire d'une liaison covalente, à
- un linker tel que défini à la revendication 2,
- un second domaine nucléotidique appelé S3, qui comprend ou consiste en un polynucléotide, ou un analogue d'un polynucléotide, et qui est capable de s'apparier en une structure double-brin avec un domaine nucléotidique complémentaire appelé S4 en milieu hydrique; et
- un linker tel que défini à la revendication 2,
- le domaine S3 ou ledit linker étant lié à l'extrémité C-terminale d'un second domaine polypeptidique, appelé DV2a, qui est caractéristique d'un DV d'une chaîne lourde ou d'une chaîne légère d'un anticorps, DV2a et le domaine polypeptidique (i) de la molécule chimérique A ou B étant caractéristiques de DV d'anticorps distincts, et
- un élément lipophile tel que défini à la revendication 5, ledit élément lipophile étant relié à DV2a par l'intermédiaire d'une liaison labile en milieu réducteur ou par l'intermédiaire d'un linker tel que défini à la revendication 2, ledit linker comportant une liaison labile en milieu réducteur ou étant lié à DV2a par l'intermédiaire d'une liaison labile en milieu réducteur,
• la deuxième molécule chimérique, respectivement B ou A, comprend ou consiste en :
- un domaine polypeptidique (i) tel que défini à la revendication 1, ce domaine et le domaine polypeptidique (i) de la première molécule chimérique étant caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
et les domaines suivants, positionnés successivement dans l'ordre suivant, à partir de l'extrémité C-terminale du domaine polypeptidique (i):
- un linker tel que défini à la revendication 2, et
- un domaine nucléotidique (ii) tel que défini à la revendication 1, appelé S2, capable de s'apparier en une structure double brin avec S1 en milieu hydrique.

8. Hémi-anticorps selon l'une quelconque des revendications 1 à 7, dans lequel :
• la première molécule chimérique, A ou B, comprend ou consiste en:
- un domaine polypeptidique (i) tel que défini à la revendication 1, et les domaines suivants, positionnés successivement dans l'ordre suivant, à partir de l'extrémité C-terminale du domaine polypeptidique (i):
- un linker tel que défini à la revendication 2,
- le domaine nucléotidique (ii) tel que défini à la revendication 1, appelé S5,
- un linker tel que défini à la revendication 2, ledit linker comportant une liaison labile en milieu réducteur ou étant lié par l'intermédiaire d'une liaison labile en milieu réducteur, à,
- un second domaine nucléotidique appelé S4, qui comprend ou consiste en un polynucléotide, ou un analogue d'un polynucléotide, et qui est capable de s'apparier en une structure double-brin avec un domaine nucléotidique complémentaire appelé S3, en milieu hydrique; et
- un linker tel que défini à la revendication 2,
- le domaine S4 ou ledit linker étant lié à l'extrémité C-terminale d'un second domaine polypeptidique, appelé DV2b, qui est caractéristique d'un DV d'une chaîne lourde ou d'une chaîne légère d'un anticorps, DV2b et le domaine polypeptidique (i) de la première molécule chimérique étant caractéristiques de DV d'anticorps distincts, et
- un élément lipophile tel que défini à la revendication 5, ledit élément lipophile étant relié à DV2b par l'intermédiaire d'une liaison labile en milieu réducteur ou par l'intermédiaire d'un linker tel que défini à la revendication 2, ledit linker comportant une liaison labile en milieu réducteur ou étant lié à DV2a par l'intermédiaire d'une liaison labile en milieu réducteur; et
• la deuxième molécule chimérique, respectivement B ou A, comprend ou consiste en :
- un domaine polypeptidique (i) tel que défini à la revendication 1, ce domaine et le domaine polypeptidique (i) de la première molécule chimérique étant caractéristiques de DV d'un même anticorps, l'un de ces domaines étant caractéristique d'un DV de la chaîne légère d'un anticorps donné et l'autre étant caractéristique d'un DV de la chaîne lourde du même anticorps,
et les domaines suivants, positionnés successivement dans l'ordre suivant, à partir de l'extrémité C-terminale du domaine polypeptidique (i):
- un linker tel que défini à la revendication 2, et
- un domaine nucléotidique (ii) tel que défini à la revendication 1, appelé S6, capable de s'apparier en une structure double brin avec S5 en milieu hydrique.

9. Hémi-anticorps selon l'une quelconque des revendications 1, 7 et 8, dans lequel ledit polynucléotide est un ADN ou un ARN.

10. Hémi-anticorps selon l'une quelconque des revendications 1, 7 et 8, dans lequel ledit analogue d'un polynucléotide est un acide nucléique peptidique (ANP), un acide nucléique bloqué (ou LNA ; Locked Nucleic Acid), un acide nucléique méthylphosphonate, ou un acide nucléique thioate.

11. Hémi-anticorps selon l'une quelconque des revendications 1 à 10, dans lequel les domaines polypeptidiques (i) sont caractéristiques de domaines DV d'un ou plusieurs anticorps dirigé(s) :
- contre un antigène présent à la surface de cellules de mammifère, ou
- contre un antigène viral, ou
- contre un antigène bactérien, ou
- contre une rétrotranscriptase.

12. Hémi-anticorps selon la revendication 11, dans lequel ledit antigène présent à la surface de cellules de mammifère est la protéine CD4, ledit antigène viral est la protéine d'enveloppe externe d'un virus VIH, ledit antigène bactérien est une protéine d'une enveloppe bactérienne, ou ladite rétrotranscriptase est une rétrotranscriptase de VIH-1 ou VIH-2.

13. Hémi-anticorps selon l'une quelconque des revendications 5 à 12, dans lequel le domaine étiquette et/ou le domaine biologiquement actif comprend ou consiste en deux ANPzymes, lesdites ANPzymes étant liées par leurs extrémités N-terminales ou C-terminales par l'intermédiaire d'un même linker.

14. Hémi-anticorps selon l'une quelconque des revendications 5, 6-8, dans lequel la liaison labile en milieu réducteur est un pont disulfure ou dans lequel la liaison persistante en milieu réducteur est une liaison établie avec un acide maléïmide.

15. Vésicule comportant un ou plusieurs hémi-anticorps tel(s) que défini(s) dans l'une quelconque des revendications 1 à 14, ladite vésicule comportant:
• un hémi-anticorps tel que défini à la revendication 7, appelé HAC_1, dans lequel
- le domaine polypeptidique (i) des molécules chimériques A et B est caractéristique d'un DV d'un anticorps dirigé contre un premier antigène présent à la surface de cellules de mammifère ou dirigé contre un antigène viral; et
- le domaine polypeptidique DV2a est caractéristique d'un DV d'un anticorps anti rétro-transcriptase; ou
• un hémi-anticorps tel que défini à la revendication 8, appelé HAC_2, dans lequel
- le domaine polypeptidique (i) des molécules chimériques A et B est caractéristique d'un DV d'un anticorps dirigé contre un deuxième antigène présent à la surface de cellules de mammifère, ou dirigé contre un antigène viral; et
- le domaine polypeptidique DV2b est caractéristique d'un DV du même anticorps que le domaine DV2a, DV2a ou DV2b étant caractéristique d'un DV de la chaîne légère d'un anticorps donné, et l'autre domaine, respectivement DV2b ou DV2a, étant caractéristique d'un DV d'une chaîne lourde du même anticorps.

16. Vésicule selon la revendication 15, ladite vésicule étant un liposome constitué de membrane de particules du virus VIH-1 ou VIH-2, dans laquelle ledit antigène viral est la protéine d'enveloppe externe d'un virus VIH, ladite anti rétro-transcriptase est une anti rétro-transcriptase de VIH-1 ou VIH-2, ou ledit deuxième antigène est la protéine CD4.

17. Particule sur laquelle sont liés, un ou plusieurs hémi-anticorps tel(s) que défini(s) dans l'une quelconque des revendications 1 à 14 par l'intermédiaire
- d'une ou plusieurs liaison(s) labile(s) en milieu réducteur; ou
- d'un ANP ou d'un acide nucléique comportant une séquence complémentaire à une séquence nucléique dudit hémi-anticorps.

18. Particule selon la revendication 17, ladite particule étant une nano particule ou Qdot.

19. Composition pharmaceutique ou thérapeutique, comprenant, consistant en ou consistant essentiellement en un ou plusieurs hémi-anticorps tel(s) que défini(s) dans l'une quelconque des revendications 1 à 14, ou une ou plusieurs vésicules telle(s) que définie(s) à la revendication 15 ou 16, ou une ou plusieurs particule(s) telle(s) que définie(s) à la revendication 17 ou 18 et, le cas échéant, un support, un diluant et/ou un véhicule pharmaceutiquement acceptable.

20. Composition selon la revendication 19, pour une utilisation comme médicament pour la prévention et/ou le traitement d'un cancer et/ou d'une maladie génétique, et/ou d'une maladie infectieuse, et/ou d'une infection bactérienne.

21. Composition pour une utilisation selon la revendication 20, dans laquelle la maladie génétique est une myopathie, et/ou la maladie infectieuse est une infection par un lentivirus, et/ou l'infection bactérienne est une infection par une bactérie résistante à des antibiotiques.

22. Composition selon la revendication 21, dans laquelle la maladie infectieuse est une infection par VIH-1 ou VIH-2.

23. Kit comprenant un ou plusieurs hémi-anticorps tel(s) que défini(s) dans l'une quelconque des revendications 1 à 14, une ou plusieurs vésicules telle(s) que définie(s) à la revendication 15 ou 16, une ou plusieurs particule(s) telle(s) que définie(s) à la revendication 17 ou 18 ou une ou plusieurs composition(s) telle(s) que définie(s) dans l'une quelconque des revendications 19 à 22, et le cas échéant, une notice d'utilisation.

24. Kit selon la revendication 23, pour une utilisation telle que définie à la revendication 20.

25. Méthode pour détecter et le cas échéant quantifier un ou plusieurs antigène(s) d'intérêt éventuellement présents dans un échantillon, ladite méthode comprenant ou consistant en les étapes suivantes :
- la mise en contact des antigènes de l'échantillon avec un ou plusieurs hémi-anticorps tel(s) que défini(s) dans l'une quelconque des revendications 1 à 14 ;
- la détection des complexes éventuellement formés entre ledit ou lesdits hémi-anticorps et un ou plusieurs antigènes de l'échantillon ;
- le cas échéant, la quantification du ou des antigènes ainsi détectés,
dans laquelle les antigènes éventuellement présents dans l'échantillon biologique ont été préalablement fixés sur un support solide.

26. Puce **caractérisée en ce qu'**elle comporte un ou plusieurs hémi-anticorps tel(s) que défini(s) dans l'une quelconque des revendications 1 à 14, ledit ou lesdits hémi-anticorps étant lié(s) sur une ou plusieurs unités d'hybridation (ou spots) de ladite puce.

27. Méthode pour détecter et le cas échéant quantifier un ou plusieurs antigène(s) d'intérêt éventuellement présent(s) dans un échantillon, ladite méthode comprenant ou consistant en les étapes suivantes :
a) la mise en contact des unités d'hybridation (ou spots) d'une puce à acide(s) nucléique(s) avec un (ou plusieurs) hémi-anticorps tel(s) que défini(s) dans l'une quelconque des revendications 1 à 14, l'hémi-anticorps étant capable de se fixer à une ou plusieurs unité(s) d'hybridation de ladite puce ;
b) la mise en contact des unités d'hybridation de la puce (ou des unités de la puce mises en contact avec l'hémi-anticorps à l'étape a) avec les protéines ou les antigènes de l'échantillon (ou plus généralement avec l'échantillon) ;
c) la détection des complexes éventuellement formés entre l'hémi-anticorps fixé sur la puce et un ou plusieurs antigène(s) de l'échantillon ;
d) le cas échéant, la quantification du ou des antigènes ainsi détectés.

28. Kit comprenant ou consistant en :
a)
- une puce à acide(s) nucléique(s) ; et
- un ou plusieurs hémi-anticorps tel(s) que défini(s) dans l'une quelconque des revendications 1 à 14 ou une ou plusieurs composition(s) telle(s) que définie(s) à la revendication 19 ; et
- et le cas échéant, une notice d'utilisation ; ou
b)
- une puce à acide(s) nucléique(s), qui comporte une ou plusieurs unités d'hybridation (ou spots) sur lesquelles un hémi-anticorps tel que défini dans l'une quelconque des revendications 1 à 14 est hybridé ; et
- et le cas échéant, une notice d'utilisation.

## Patentansprüche

1. Chimäres Molekül, bezeichnet als "Antikörperhälfte", **dadurch gekennzeichnet, dass** es zwei chimäre Moleküle A und B umfasst oder aus diesen besteht, die jeweils umfassen oder bestehen aus:
(i) einer Polypeptid-Domäne, die für eine variable Domäne (oder VD) einer schweren Kette oder einer leichten Kette eines Antikörpers charakteristisch ist, wobei diese Polypeptid-Domäne an einem Ende in den chimären Molekülen A und B positioniert ist, wobei die Polypeptid-Domäne (i) eines der beiden chimären Moleküle A oder B für eine VD einer leichten Kette eines Antikörpers charakteristisch ist und die Polypeptid-Domäne (i) des anderen chimären Moleküls, respektive B
oder A, für eine VD einer schweren Kette eines Antikörpers charakteristisch ist; und
(ii) einer einzelsträngigen Nucleotid-Domäne, die aus einem Polynucleotid oder einem Polynucleotid-Analogon besteht, wobei die Nucleotid-Domäne von A und die von B sich zu einer Doppelstrangstruktur im wässrigen Medium verbinden können;
wobei die Polypeptid-Domäne (i) und die Nucleotid-Domäne (ii) eines jeden chimären Moleküls A und B durch eine kovalente Bindung verbunden sind und die Domäne (ii) des chimären Moleküls A und die des chimären Moleküls B in einer Doppelstrangstruktur im wässrigen Medium verbunden sind.

2. Antikörperhälfte nach Anspruch 1,
wobei die Polypeptid-Domäne (i) und die Nucleotid-Domäne (ii) eines chimären Moleküls A oder B durch ein oder mehrere Bindungsmoleküle (oder Linker) verbunden sind, wobei der oder die Linker eine Hydrogencarbonat-Kette umfassen oder aus dieser bestehen, und ein oder mehrere Elemente umfassen oder aus diesen bestehen, die ausgewählt sind aus einem Peptid oder einem Polypeptid, einem Polynucleotid oder einem Polynucleotid-Analogon, einer Polycarbonat-, Ethylendiamin-, Polylysin-, Beta-Alanin-Kette und einem Zucker.

3. Antikörperhälfte nach Anspruch 1 oder 2, wobei:
- in dem chimären Molekül A das C-terminale Ende der Polypeptid-Domäne (i) kovalent mit dem 3'-Ende einer Nucleinsäure oder mit dem C-terminalen Ende eines Polynucleotid-Analogons verbunden ist, das aus einer Peptid-Nucleinsäure (PNA) besteht; und/oder
- in dem chimären Molekül B das C-terminale Ende der Polypeptid-Domäne (i) kovalent mit dem 5'-Ende einer Nucleinsäure oder mit dem N-terminalen Ende eines Polynucleotid-Analogons verbunden ist, das aus einer Peptid-Nucleinsäure (PNA) besteht.

4. Antikörperhälfte nach Anspruch 3, wobei:
- in dem chimären Molekül A die kovalente Bindung durch die NH₂-Gruppe der Seitenkette eines Lysins hergestellt wird, das mit dem C-terminalen Ende der PNA durch eine Peptidbindung verbunden ist; und/oder
- in dem chimären Molekül B die kovalente Bindung durch eine Peptid- oder Amidbindung mit einer der NH₂-Gruppen eines Lysins hergestellt wird, wobei das Lysin durch seine COOH-Gruppe mit dem N-terminalen Ende der PNA verbunden ist.

5. Antikörperhälfte nach einem der Ansprüche 1 bis 4, wobei das chimäre Molekül A und/oder das chimäre Molekül B ferner ein oder mehrere Elemente umfassen, die ausgewählt sind aus:
• einer Markierungsdomäne und/oder einer biologisch aktiven Domäne, die ein Polynucleotid oder ein Analogon ist, das eine Markierungssequenz (oder Tag-Sequenz) und/oder eine biologisch aktive Sequenz, die die Expression der Ziel-Gene zu blockieren erlaubt, umfasst oder aus diesen besteht, wobei das Polynucleotid oder das Analogon aus den folgenden Elementen ausgewählt ist:
- einer Nucleinsäure, einer Antisense-Nucleinsäure oder einer Nucleinsäure mit einer Endonuclease-Aktivität;
- einer PNA, einer Antisense-PNA oder einer PNA mit einer Endonuclease-Aktivität (PNAzym),
- einer LNA, einer Methylphosphonat-Nucleinsäure, einer Methylsulfonat-Nucleinsäure, einer modifizierten Nuclein- oder Desoxyribonucleinsäure, einem DNAzym, einem RNAzym oder einem PNAzym, einer microRNA (miRNA), einer kombinierten Sequenz aus PNA/Nucleinsäure oder PNA/Peptid, einem DNA/PNAzym-Dimer; und
- einer Kombination aus wenigstens zwei dieser Elemente,
wobei die Markierungsdomäne oder die biologisch aktive Domäne mit dem freien Ende der Nucleotid-Domäne (ii) des chimären Moleküls A oder B oder mit dem freien Ende eines mit der Nucleotid-Domäne (ii) des chimären Moleküls A oder B verbundenen Linkers verbunden ist, wobei der Linker so wie in Anspruch 2 ausgeführt ist, und die Bindung der Markierungsdomäne oder der biologisch aktiven Domäne mittels einer labilen Verbindung im Reduktionsmedium oder mittels einer persistenten Verbindung im Reduktionsmedium erfolgt; und
• einem lipophilen Element, das ausgewählt ist aus einer Sequenz, einer chemischen Gruppe oder einer lipophilen chemischen Verbindung, das eine oder mehrere Gruppen und/oder aromatische Verbindungen umfasst, wobei das lipophile Element an dem freien Ende des chimären Moleküls A oder B positioniert ist.

6. Antikörperhälfte nach einem der Ansprüche 1 bis 5, wobei:
• das erste chimäre Molekül A oder B umfasst oder besteht aus:
- einer Polypeptid-Domäne (i) wie in Anspruch 1 definiert und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert,
- eine Nucleotid-Domäne (ii) wie in Anspruch 1 definiert,
- ein Linker wie in Anspruch 2 definiert, der mittels einer labilen Verbindung im Reduktionsmedium oder mittels einer persistenten Verbindung im Reduktionsmedium verbunden ist mit
- einer Markierungsdomäne oder der biologisch aktiven Domäne wie in Anspruch 5 definiert, und
- ein lipophiles Element wie in Anspruch 5 definiert; und
• das zweite chimäre Molekül, respektive B oder A umfasst oder besteht aus:
- einer Polypeptid-Domäne (i) wie in Anspruch 1 definiert, wobei diese Domäne und die Polypeptid-Domäne (i) des ersten chimären Moleküls für eine VD ein und desselben Antikörpers charakteristisch sind, wobei eine der Domänen für eine VD der leichten Kette eines gegebenen Antikörpers und die andere für eine VD der schweren Kette desselben Antikörpers charakteristisch ist,
und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert, und
- eine Nucleotid-Domäne (ii) wie in Anspruch 1 definiert.

7. Antikörperhälfte nach einem der Ansprüche 1 bis 6, wobei:
• das erste chimäre Molekül A oder B umfasst oder besteht aus:
- einer Polypeptid-Domäne (i) wie in Anspruch 1 definiert, und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert,
- eine Nucleotid-Domäne (ii) wie in Anspruch 1 definiert, bezeichnet als S1,
- ein Linker wie in Anspruch 2 definiert, der mittels einer labilen Verbindung im Reduktionsmedium verbunden ist mit
- einer Markierungsdomäne oder einer biologisch aktiven Domäne wie in Anspruch 5 definiert, die mittels einer kovalenten Bindung verbunden ist mit
- einem Linker wie in Anspruch 2 definiert,
- einer zweiten Nucleotid-Domäne, bezeichnet als S3, die ein Polynucleotid oder ein Polynucleotid-Analogon umfasst oder aus diesem besteht und die sich mit einer komplementären Nucleotid-Domäne, bezeichnet als S4, in einer Doppelstrangstruktur im wässrigen Medium verbinden kann; und
- ein Linker wie in Anspruch 2 definiert,
- wobei die S3-Domäne oder der Linker mit dem C-terminalen Ende einer zweiten Polypeptid-Domäne, bezeichnet als VD2a, verbunden sind, die für eine VD einer schweren Kette oder einer leichten Kette eines Antikörpers charakteristisch ist, wobei VD2a und die Polypeptid-Domäne (i) des chimären Moleküls A oder B für die VD eines separaten Antikörpers charakteristisch sind, und
- ein lipophiles Element wie in Anspruch 5 definiert, wobei das lipophile Element mittels einer labilen Verbindung im Reduktionsmedium oder mittels eines Linkers wie in Anspruch 2 definiert mit der VD2a verbunden ist, wobei der Linker eine labile Verbindung im Reduktionsmedium aufweist oder mittels einer labilen Verbindung im Reduktionsmedium mit der VD2a verbunden ist,
• das zweite chimäre Molekül, respektive B oder A, umfasst oder besteht aus:
- einer Polypeptid-Domäne (i) wie in Anspruch 1 definiert, wobei diese Domäne und die Polypeptid-Domäne (i) des ersten chimären Moleküls für die VD ein und desselben Antikörpers charakteristisch sind, wobei eine der Domänen für eine VD der leichten Kette eines gegebenen Antikörpers und die andere für eine VD der schweren Kette desselben Antikörpers charakteristisch ist,
und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert, und
- eine Nucleotid-Domäne (ii) wie in Anspruch 1 definiert, bezeichnet als S2, die sich mit S1 zu einer Doppelstrangstruktur im wässrigen Medium verbinden kann.

8. Antikörperhälfte nach einem der Ansprüche 1 bis 7, wobei:
• das erste chimäre Molekül A oder B umfasst oder besteht aus:
- einer Polypeptid-Domäne (i) wie in Anspruch 1 definiert, und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert,
- eine Nucleotid-Domäne (ii) wie in Anspruch 1 definiert, bezeichnet als S5,
- ein Linker wie in Anspruch 2 definiert, wobei der Linker eine labile Verbindung im Reduktionsmedium aufweist oder mittels einer labilen Verbindung im Reduktionsmedium verbunden ist mit
- einer zweiten Nucleotid-Domäne, bezeichnet als S4, die ein Polynucleotid oder ein Polynucleotid-Analogon umfasst oder aus diesem besteht und die sich mit einer komplementären Nucleotid-Domäne, bezeichnet als S3, zu einer Doppelstrangstruktur im wässrigen Medium verbinden kann; und
- einem Linker wie in Anspruch 2 definiert,
- wobei die S4-Domäne oder der Linker mit dem C-terminalen Ende einer zweiten Polypeptid-Domäne, bezeichnet als VD2b, verbunden ist, die für eine VD einer schweren Kette oder einer leichten Kette eines Antikörpers charakteristisch ist, wobei VD2b und die Polypeptid-Domäne (i) des chimären Moleküls für die VD eines separaten Antikörpers charakteristisch sind, und
- ein lipophiles Element wie in Anspruch 5 definiert, wobei das lipophile Element mittels einer labilen Verbindung im Reduktionsmedium oder mittels eines Linkers wie in Anspruch 2 definiert mit der VD2b verbunden ist, wobei der Linker eine labile Verbindung im Reduktionsmedium aufweist, oder mittels einer labilen Verbindung im Reduktionsmedium mit der VD2b verbunden ist; und
• das zweite chimäre Molekül, respektive B oder A, umfasst oder besteht aus:
- einer Polypeptid-Domäne (i) wie in Anspruch 1 definiert, wobei diese Domäne und die Polypeptid-Domäne (i) des ersten chimären Moleküls für die VD ein und desselben Antikörpers charakteristisch sind, wobei eine der Domänen für eine VD der leichten Kette eines gegebenen Antikörpers und die andere für eine VD der schweren Kette desselben Antikörpers charakteristisch ist,
und den folgenden Domänen, die ausgehend von dem C-terminalen Ende der Polypeptid-Domäne (i) nacheinander in der folgenden Reihenfolge positioniert sind:
- ein Linker wie in Anspruch 2 definiert, und
- eine Nucleotid-Domäne (ii) wie in Anspruch 1 definiert, bezeichnet als S6, die sich mit S5 zu einer Doppelstrangstruktur im wässrigen Medium verbinden kann.

9. Antikörperhälfte nach einem der Ansprüche 1, 7 und 8,
wobei das Polynucleotid eine DNA oder eine RNA ist.

10. Antikörperhälfte nach einem der Ansprüche 1, 7 und 8,
wobei das Polynucleotid-Analogon eine Peptid-Nucleinsäure (PNA), eine verbrückte Nucleinsäure (LNA, Locked Nucleic Acid), eine Methylphosphonat-Nucleinsäure oder eine Thioat-Nucleinsäure ist.

11. Antikörperhälfte nach einem der Ansprüche 1 bis 10,
wobei die Polypeptid-Domänen (i) für VD-Domänen eines oder mehrerer Antikörper charakteristisch sind, die
- gegen ein auf der Oberfläche von Zellen von Säugetieren vorhandenes Antigen, oder
- gegen ein virales Antigen, oder
- gegen ein bakterielles Antigen, oder
- gegen eine reverse Transkriptase
gerichtet sind.

12. Antikörperhälfte nach Anspruch 11,
wobei das auf der Oberfläche von Zellen von Säugetieren vorhandene Antigen das CD4-Protein ist, das virale Antigen das äußere Hüllprotein eines HI-Virus ist, das bakterielle Antigen ein Protein einer Bakterienhülle ist oder die reverse Transkriptase eine reverse Transkriptase von HIV-1 oder HIV-2 ist.

13. Antikörperhälfte nach einem der Ansprüche 5 bis 12,
wobei die Markierungsdomäne und/oder die biologisch aktive Domäne zwei ANPzyme umfassen oder aus diesen bestehen, wobei die ANPzyme durch ihre N-terminalen oder C-terminalen Enden mittels ein und desselben Linkers verbunden sind.

14. Antikörperhälfte nach einem der Ansprüche 5, 6 bis 8,
wobei die labile Verbindung im Reduktionsmedium eine Disulfidbrücke ist oder wobei die persistente Verbindung im Reduktionsmedium eine mit einer Maleimidsäure hegestellte Verbindung ist.

15. Vesikel mit einer oder mehreren Antikörperhälften wie in einem der Ansprüche 1 bis 14 definiert, wobei die Vesikel umfasst:
• eine Antikörperhälfte wie in Anspruch 7 definiert, bezeichnet als HAC_1, wobei
- die Polypeptid-Domäne (i) der chimären Moleküle A und B charakteristisch ist für eine VD eines Antikörpers, der gegen ein erstes auf der Oberfläche von Zellen von Säugetieren vorhandenes Antigen gerichtet ist oder der gegen ein virales Antigen gerichtet ist; und
- die Polypeptid-Domäne VD2a charakteristisch ist für eine VD eines die reverse Transkriptase hemmenden Antikörpers; oder
• eine Antikörperhälfte wie in Anspruch 8 definiert, bezeichnet als HAC_2, wobei
- die Polypeptid-Domäne (i) der chimären Moleküle A und B charakteristisch ist für eine VD eines Antikörpers, der gegen ein zweites auf der Oberfläche von Zellen von Säugetieren vorhandenes Antigen gerichtet ist oder der gegen ein virales Antigen gerichtet ist; und
- die Polypeptid-Domäne VD2b für eine VD desselben Antikörpers wie die Domäne VD2a charakteristisch ist, wobei VD2a oder VD2b für eine VD der leichten Kette eines gegebenen Antikörpers charakteristisch ist und die andere Domäne, respektive VD2b oder VD2a, für eine VD einer schweren Kette desselben Antikörpers charakteristisch ist.

16. Vesikel nach Anspruch 15,
wobei die Vesikel ein Liposom ist, das aus einer Partikelmembran des HI-Virus Typ 1 oder Typ 2 gebildet ist, bei dem das virale Antigen das äußere Hüllprotein eines HI-Virus ist, der reverse Transkriptase-Hemmer ein reverse Transkriptase-Hemmer von HIV-1 oder HIV-2 ist oder das zweite Antigen das CD4-Protein ist.

17. Partikel, auf dem eine oder mehrere Antikörperhälften wie in einem der Ansprüche 1 bis 14 definiert gebunden sind, mittels
- einer oder mehreren labilen Verbindungen im Reduktionsmedium; oder
- einer PNA oder einer Nucleinsäure mit einer zu einer Nucleinsäuresequenz der Antikörperhälfte komplementären Sequenz.

18. Partikel nach Anspruch 17,
wobei das Partikel ein Nanopartikel oder Quantenpunkt QD ist.

19. Pharmazeutische oder therapeutische Zusammensetzung,
die eine oder mehrere Antikörperhälften wie in einem der Ansprüche 1 bis 14 definiert oder eine oder mehrere Vesikel wie in Anspruch 15 oder 16 definiert oder ein oder mehrere Partikel wie in Anspruch 17 oder 18 definiert und gegebenenfalls einen pharmazeutisch verträglichen Träger, Verdünner und/oder Hilfsstoff umfasst, aus diesen besteht oder im Wesentlichen aus diesen besteht.

20. Zusammensetzung nach Anspruch 19,
zur Verwendung als Medikament zur Vorbeugung und/oder Behandlung eines Karzinoms und/oder einer Erbkrankheit und/oder einer Infektionskrankheit und/oder einer bakteriellen Erkrankung.

21. Zusammensetzung zur Verwendung nach Anspruch 20,
wobei die Erbkrankheit eine Myopathie ist und/oder die Infektionskrankheit eine Infektion durch einen Lentivirus ist und/oder die bakterielle Erkrankung eine Infektion durch ein antibiotikaresistentes Bakterium ist.

22. Zusammensetzung nach Anspruch 21,
wobei die Infektionskrankheit eine HIV-1- oder HIV-2-Infektion ist.

23. Kit mit einer oder mehreren Antikörperhälften wie in einem der Ansprüche 1 bis 14 definiert,
einer oder mehreren Vesikel wie in Anspruch 15 oder 16 definiert, einem oder mehreren Partikel wie in Anspruch 17 oder 18 definiert oder einer oder mehreren Zusammensetzungen wie in einem der Ansprüche 19 bis 22 definiert und gegebenenfalls einer Gebrauchsinformation.

24. Kit nach Anspruch 23,
zur Verwendung wie in Anspruch 20 definiert.

25. Verfahren zum Nachweis und gegebenenfalls zur Quantifizierung einer oder mehrerer interessierender, in einer Probe vorhandener Antikörperhälften, wobei das Verfahren die folgenden Schritte umfasst bzw. aus den folgenden Schritten besteht:
- Inkontaktbringen der Antigene der Probe mit einer oder mehreren Antikörperhälften wie in einem der Ansprüche 1 bis 14 definiert;
- Nachweis der eventuell zwischen der bzw. den Antikörperhälften und einem oder mehreren Antigenen der Probe gebildeten Komplexverbindungen;
- gegebenenfalls Quantifizierung des oder der auf diese Weise nachgewiesenen Antigene,
wobei die eventuell in der biologischen Probe vorhandenen Antigene zuvor auf einem festen Träger fixiert worden sind.

26. Chip, **dadurch gekennzeichnet, dass** er eine oder mehrere Antikörperhälften wie in einem der Ansprüche 1 bis 14 definiert umfasst, wobei die Antikörperhälfte bzw. -hälften auf einer oder mehreren Hybridisierungseinheiten (oder Spots) des Chips gebunden sind.

27. Verfahren zum Nachweis und gegebenenfalls zur Quantifizierung eines oder mehrerer interessierender, in einer Probe vorhandener Antigene, wobei das Verfahren die folgenden Schritte umfasst bzw. aus den folgenden Schritten besteht:
a) Inkontaktbringen der Hybridisierungseinheiten (oder Spots) eines Nucleinsäure-Chips mit einer oder mehreren Antikörperhälften wie in einem der Ansprüche 1 bis 14 definiert, wobei die Antikörperhälfte an einer oder mehreren Hybridisierungseinheiten des Chips fixiert werden kann;
b) Inkontaktbringen der Hybridisierungseinheiten des Chips (oder der mit der Antikörperhälfte in Schritt a) in Kontakt gebrachten Einheiten des Chips) mit den Proteinen oder den Antigenen der Probe (oder allgemein mit der Probe);
c) Nachweis der eventuell zwischen der auf dem Chip fixierten Antikörperhälfte und einem oder mehreren Antigenen der Probe gebildeten Komplexverbindungen;
d) gegebenenfalls Quantifizierung des oder der auf diese Weise nachgewiesenen Antigene.

28. Kit umfassend oder bestehend aus:
- einem Nucleinsäure-Chip; und
- einer oder mehreren Antikörperhälften wie in einem der Ansprüche 1 bis 14 definiert oder einer oder mehreren Zusammensetzungen wie in Anspruch 19 definiert; und
- gegebenenfalls einer Gebrauchsinformation; oder
- einem Nucleinsäure-Chip, der eine oder mehrere Hybridisierungseinheiten (oder Spots) umfasst, auf denen eine Antikörperhälfte wie in einem der Ansprüche 1 bis 14 definiert hybridisiert wird; und
- gegebenenfalls einer Gebrauchsinformation.

## Claims

1. A chimeric molecule termed a "half-antibody", **characterized in that** it comprises or consists of two chimeric molecules A and B, each comprising or consisting of:
(i) a characteristic polypeptide domain of a variable domain (or VD) of a heavy chain or of a light chain of an antibody, this polypeptide domain being positioned at one end in the chimeric molecules A and B, the polypeptide domain (i) of one of the two chimeric molecules, A or B, being characteristic of a VD of a light chain of an antibody and the polypeptide domain (i) of the other chimeric molecule, respectively B or A, being characteristic of a VD of a heavy chain of an antibody; and
(ii) a single-stranded nucleotide domain consisting of a polynucleotide, or an analog of a polynucleotide, the nucleotide domain of A and that of B being capable of pairing into a double-stranded structure in a hydric medium;
the polypeptide domain (i) and the nucleotide domain (ii) of each of the chimeric molecules A and B being bonded via a covalent bond, and the domain (ii) of the chimeric molecule A and that of the chimeric molecule B being paired into a double-stranded structure in a hydric medium.

2. A half-antibody according to claim 1, in which the polypeptide domain (i) and the nucleotide domain (ii) of one chimeric molecule A or B are linked by one or more binding molecule(s) (or linker(s)), said linker(s) comprising orconsisting of a hydrocarbon chain, and comprising or consisting of one or more element(s) selected from a peptide or a polypeptide, a polynucleotide or an analog of a polynucleotide, a polycarbon chain, ethylenediamine, polylysine, beta-alanine, and a sugar.

3. A half-antibody according to claim 1 or 2, in which:
- in the chimeric molecule A, the C-terminal end of the polypeptide domain (i) is covalently bonded to the 3' end of a nucleic acid or to the C-terminal end of an analog of a polynucleotide consisting of a peptide nucleic acid (PNA); and/or
- in the chimeric molecule B, the C-terminal end of the polypeptide domain (i) is covalently bonded to the 5' end of the nucleic acid or to the N-terminal end of an analog of a polynucleotide consisting of a peptide nucleic acid (PNA).

4. A half-antibody according to claim 3, in which:
- in the chimeric molecule A, the covalent bond is achieved via the NH₂ group of the side chain of a lysine bonded to the C-terminal end of said PNA via a peptide linkage; and/or
- in the chimeric molecule B, the covalent bond is achieved via a peptide linkage or amide with one of the NH₂ groups of a lysine, said lysine being bonded, via its COOH group, to the N-terminal end of said PNA.

5. A half-antibody according to any one of claims 1 to 4, in which the chimeric molecule A and/or the chimeric molecule B further comprise(s) one or more element(s) selected from:
• a tag domain and/or a biologically active domain, which is a polynucleotide or an analog comprising or consisting of a tag sequence and/or a biologically active sequence that can be used to block the expression of target genes, said polynucleotide or said analog being selected from the following elements:
- a nucleic acid, an antisense nucleic acid or a nucleic acid with endonuclease activity;
- a PNA, an antisense PNA or a PNA with endonuclease activity (PNAzyme),
- a LNA, a methylphosphonate nucleic acid, a nucleic acid methylsulfonate, a modified nucleic acid or deoxynucleic acid, a DNAzyme, a RNAzyme or a PNAzyme, a micro RNA (miRNA), a mixed PNA/nucleic acid or PNA/peptide sequence, or a DNA/PNA-zyme dimer; and
- a combination of at least two of these elements,
said tag domain or the biologically active domain being bonded to the free end of the nucleotide domain (ii) of the chimeric molecule A or B or to the free end of a linker bonded to the nucleotide domain (ii) of the chimeric molecule A or B, said linker being as defined in claim 2, and bonding of said tag domain or of said biologically active domain occurring via a bond that is labile in a reducing medium, or via a bond that persists in a reducing medium; and
• a lipophilic element chosen among a sequence, a chemical group or a lipophilic chemical compound, which comprises one or more aromatic compound(s) and/or group(s), said lipophilic element being positioned at the free end of the chimeric molecule A or B.

6. A half-antibody according to any one of claims 1 to 5, in which:
• the first chimeric molecule, A or B, comprises or consists of:
- a polypeptide domain (i) as defined in claim 1, and the following domains, positioned in succession in the following order, starting from the C-terminal end of the polypeptide domain (i):
- a linker as defined in claim 2,
- a nucleotide domain (ii) as defined in claim 1,
- a linker as defined in claim 2, bonded via a bond that is labile in a reducing medium or via a bond that persists in a reducing medium, to
- a tag domain or a biologically active domain as defined in claim 5, and
- a lipophilic element as defined in claim 5; and
• the second chimeric molecule, respectively B or A, comprises or consists of:
- a polypeptide domain (i) as defined in claim 1, this domain and the polypeptide domain (i) of the first chimeric molecule being characteristic of VDs of the same antibody, one of these domains being characteristic of a VD of the light chain of a given antibody and the other being characteristic of a VD of the heavy chain of the same antibody,
and the following domains, positioned in succession in the following order, starting from the C-terminal end of the polypeptide domain (i):
- a linker as defined in claim 2, and
- a nucleotide domain (ii) as defined in claim 1.

7. A half-antibody according to any one of claims 1 to 6, in which:
• the first chimeric molecule, A or B, comprises or consists of:
- a polypeptide domain (i) as defined in claim 1, and the following domains, positioned in succession in the following order, starting from the C-terminal end of polypeptide domain (i):
- a linker as defined in claim 2,
- a nucleotide domain (ii) as defined in claim 1, termed S1
- a linker as defined in claim 2, bonded via a bond that is labile in a reducing medium to:
- a tag domain or a biologically active domain as defined in claim 5, bonded via a covalent bond, to
- a linker as defined in claim 2,
- a second nucleotide domain termed S3, which comprises or consists of a polynucleotide, or an analog of a polynucleotide, and which is capable of pairing into a double-stranded structure with a complementary nucleotide domain termed S4 in an aqueous medium; and
- a linker as defined in claim 2,
- the domain S3 or said linker being bonded to the C-terminal end of a second polypeptide domain, termed VD2a, which is characteristic of a VD of a heavy chain or of a light chain of an antibody, VD2a and the polypeptide domain (i) of the chimeric molecule A or B being characteristic of VDs of distinct antibodies, and
- a lipophilic element as defined in claim 5, said lipophilic element being bonded to VD2a via a bond that is labile in a reducing medium or via a linker as defined in claim 2, said linker comprising a bond that is labile in a reducing medium or being bonded to VD2a via a bond that is labile in a reducing medium,
• the second chimeric molecule, respectively B or A, comprises or consists of:
- a polypeptide domain (i) as defined in claim 1, this domain and the polypeptide domain (i) of the first chimeric molecule being characteristic of VDs of the same antibody, one of these domains being characteristic of a VD of the light chain of a given antibody and the other being characteristic of a VD of the heavy chain of the same antibody,
and the following domains, positioned in succession in the following order, starting from the C-terminal end of the polypeptide domain (i):
- a linker as defined in claim 2, and
- a nucleotide domain (ii) as defined in claim 1, termed S2, capable of pairing into a double-stranded structure with S1 in an aqueous medium.

8. A half-antibody according to any one of claims 1 to 7, in which:
• the first chimeric molecule, A or B, comprises or consists of:
- a polypeptide domain (i) as defined in claim 1, and the following domains, positioned in succession in the following order, starting from the C-terminal end of the polypeptide domain (i):
- a linker as defined in claim 2,
- the nucleotide domain (ii) as defined in claim 1, termed S5,
- a linker as defined in claim 2, said linker comprising a bond that is labile in a reducing medium or being bonded via a bond that is labile in a reducing medium, to:
- a second nucleotide domain termed S4, which comprises or consists of a polynucleotide, or an analog of a polynucleotide, and which is capable of pairing into a double-stranded structure with a complementary nucleotide domain termed S3, in an aqueous medium; and
- a linker as defined in claim 2,
- the domain S4 or said linker being bonded to the C-terminal end of a second polypeptide domain, termed VD2b, which is characteristic of a VD of a heavy chain or of a light chain of an antibody, VD2b and the polypeptide domain (i) of the first chimeric molecule being characteristic of VDs of distinct antibodies, and
- a lipophilic element as defined in claim 5, said lipophilic element being bonded to VD2b via a bond that is labile in a reducing medium or via a linker as defined in claim 2, said linker comprising a bond that is labile in a reducing medium or being bonded to VD2a via a bond that is labile in a reducing medium; and
• the second chimeric molecule, respectively B or A, comprises or consists of:
- a polypeptide domain (i) as defined in claim 1, this domain and the polypeptide domain (i) of the first chimeric molecule being characteristic of VDs of the same antibody, one of these domains being characteristic of a VD of the light chain of a given antibody and the other being characteristic of a VD of the heavy chain of the same antibody,
and the following domains, positioned in succession in the following order, starting from the C-terminal end of the polypeptide domain (i):
- a linker as defined in claim 2, and
- a nucleotide domain (ii) as defined in claim 1, termed S6, which is capable of pairing into a double-stranded structure with S5 in an aqueous medium.

9. A half-antibody according to any one of claims 1, 7 and 8, in which said polynucleotide is a DNA or a RNA.

10. A half-antibody according to any one of claims 1, 7 and 8, in which said analog of a polynucleotide is a peptide nucleic acid (PNA), a locked nucleic acid (LNA), a methylphosphonate nucleic acid or a thioate nucleic acid.

11. A half-antibody according to any one of claims 1 to 10, in which the polypeptide domains (i) are characteristic of VD domains of one or more antibodies directed:
- against an antigen present on the surface of mammalian cells, or
- against a viral antigen, or
- against a bacterial antigen, or
- against a retrotranscriptase.

12. A half-antibody according to claim 11, in which said antigen present on the surface of mammalian cells is the CD4 protein, said viral antigen is the outer envelope protein of a HIV virus, said bacterial antigen is a bacterial envelope protein, or said retrotranscriptase is a retrotranscriptase of HIV-1 or HIV-2.

13. A half-antibody according to any one of claims 5 to 12, in which the tag domain and/or the biologically active domain comprises or consists of two PNAzymes, said PNAzymes being bonded via their N-terminal or C-terminal ends via the same linker.

14. A half-antibody according to any one of claims 5, 6-8, in which the bond that is labile in a reducing medium is a disulfide bridge or in which the bond that persists in a reducing medium is a bond established with a maleimide acid.

15. A vesicle comprising one or more half-antibodies as defined in any one of claims 1 to 14, said vesicle comprising:
• a half-antibody as defined in claim 7, termed HAB_1, in which
- the polypeptide domain (i) of the chimeric molecules A and B is characteristic of a VD of an antibody directed against a first antigen present on the surface of mammalian cells or directed against a viral antigen; and
- the polypeptide domain VD2a is characteristic of a VD of an anti-retrotranscriptase antibody; or
• a half-antibody as defined in claim 8, termed HAB_2, in which
- the polypeptide domain (i) of the chimeric molecules A and B is characteristic of a VD of an antibody directed against a second antigen present on the surface of mammalian cells, or directed against a viral antigen; and
- the polypeptide domain VD2b is characteristic of a VD of the same antibody as the VD2a domain, VD2a or VD2b being characteristic of a VD of the light chain of a given antibody, and the other domain, respectively VD2b or VD2a, being characteristic of a VD of a heavy chain of the same antibody.

16. A vesicle according to claim 15, said vesicle being a liposome consisting of a membrane from particles of the HIV-1 or HIV-2 virus, in which said viral antigen is the outer envelope protein of a HIV virus, said anti-retrotranscriptase is a retrotranscriptase of HIV-1 or HIV-2, or said second antigen is the CD4 protein.

17. A particle onto which one or more half-antibodies as defined in any one of claims 1 to 14 via
- one or more bond(s) labile in a reducing medium; or
- a PNA or a nucleic acid comprising a sequence that is complementary to a nucleic sequence of said half-antibody.

18. A particle according to claim 17, said particle being a nanoparticle or Q-dot.

19. A pharmaceutical or therapeutic composition comprising, consisting or essentially consisting of one or more half-antibodies as defined in any one of claims 1 to 14, or one or more vesicle(s) as defined in claim 15 or 16, or one or more particle(s) as defined in claim 17 or 18 and, if appropriate, a support, a diluent and/or a pharmaceutically acceptable vehicle.

20. A composition according to claim 19, for use as a drug for the prevention and/or treatment of a cancer and/or a genetic disease, and/or an infectious disease, and/or a bacterial infection.

21. A composition for use according to claim 20, in which the genetic disease is a myopathy, and/or the infectious disease is an infection by a lentivirus, and/or the bacterial infection is an infection by a bacterium that is resistant to antibiotics.

22. A composition according to claim 21, in which the infectious disease is an infection by HIV-1 or HIV-2.

23. A kit comprising one or more half-antibodies as defined in any one of claims 1 to 14, one or more vesicle(s) as defined in claim 15 or 16, one or more particle(s) as defined in claim 17 or 18 or one or more composition(s) as defined in any one of claims 19 to 22, and if appropriate, instructions for use.

24. A kit according to claim 23, for use as defined in claim 20.

25. A method for detecting and, if appropriate, quantifying one or more antigen(s) of interest that may be present in a sample, said method comprising or consisting of the following steps:
- bringing antigens of the sample into contact with one or more half-antibodies as defined in any one of claims 1 to 14;
- detecting any complexes that may be formed between said half-antibody or antibodies and one or more antigen(s) of the sample;
- if appropriate, quantifying the antigen or antigens detected thereby;
in which the antigens that may be present in the biological sample have been fixed onto a solid support.

26. A chip **characterized in that** it comprises one or more half-antibodies as defined in any one of claims 1 to 14, said half-antibody or half-antibodies being bonded to one or more hybridization units (or spots) of said chip.

27. A method for detecting and, if appropriate, quantifying one or more antigen(s) of interest that may be present in a sample, said method comprising or consisting of the following steps:
a) bringing hybridization units (or spots) of a nucleic acid chip into contact with one (or more) half-antibodies as defined in any one of claims 1 to 14, the half-antibody being capable of becoming attached to one or more hybridization unit(s) of said chip;
b) bringing hybridization units of the chip (or the hybridization units that have been brought into contact with the half-antibody in step a) into contact with the proteins or antigens of the sample (or more generally with the sample);
c) detecting complexes that may be formed between the half-antibody attached to the chip and one or more antigen(s) of the sample;
d) if appropriate, quantifying the antigen(s) detected thereby.

28. A kit comprising or consisting of:
a)
- a nucleic acid chip; and
- one or more half-antibodies as defined in any one of claims 1 to 14 or one or more composition(s) as defined in claim 19; and
- if appropriate, instructions for use; or
b)
- a nucleic acid chip that comprises one or more hybridization units (or spots) on which a half-antibody as defined in any one of claims 1 to 14 has been hybridized; and
- if appropriate, instructions for use.
